(19) Europäisches Patentamt
European Patent Office
Office européen des brevets

(11) EP 3 054 988 B1

(12) # FASCICULE DE BREVET EUROPEEN

(45) Date de publication et mention de la délivrance du brevet:
**07.11.2018 Bulletin 2018/45**

(21) Numéro de dépôt: **14790240.7**

(22) Date de dépôt: **09.10.2014**

(51) Int Cl.:
***A61K 47/69*** *(2017.01)* ***A61K 31/155*** *(2006.01)*
***A61K 31/7048*** *(2006.01)* ***A61K 31/722*** *(2006.01)*
***A61P 31/10*** *(2006.01)*

(86) Numéro de dépôt international:
**PCT/FR2014/052569**

(87) Numéro de publication internationale:
**WO 2015/052448 (16.04.2015 Gazette 2015/15)**

(54) **COMPOSITION ANTIPARASITAIRE ET/OU ANTIFONGIQUE COMPRENANT DU CHITOSANE HYDROPHOBISÉ**

ANTIPARASITÄRE UND/ODER ANTIFUNGALE ZUSAMMENSETZUNG MIT HYDROPHOBISIERTEM CHITOSAN

ANTIPARASITIC AND/OR ANTIFUNGAL COMPOSITION COMPRISING HYDROPHOBISED CHITOSAN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.10.2013 FR 1359777**

(43) Date de publication de la demande:
**17.08.2016 Bulletin 2016/33**

(73) Titulaires:
- **Centre National de la Recherche Scientifique (C.N.R.S.)**
  **75016 Paris (FR)**
- **Université Paris-Sud 11**
  **91400 Orsay (FR)**

(72) Inventeurs:
- **BOUCHEMAL, Kawthar**
  **F-91120 Palaiseau (FR)**
- **BORIES, Christian**
  **F-94100 Saint Maur des Fossés (FR)**
- **LOISEAU, Philippe**
  **F-91190 Gif sur Yvette (FR)**
- **VAUTHIER-HOLTZSCHERER, Christine**
  **F-91190 Gif sur Yvette (FR)**
- **POMEL, Sébastien**
  **F-91940 Les Ulis (FR)**
- **PRADINES, Bénédicte**
  **F-92296 Chatenay-Malabry Cedex (FR)**

(74) Mandataire: **Lavoix**
**2, place d'Estienne d'Orves**
**75441 Paris Cedex 09 (FR)**

(56) Documents cités:
- **KAMBIZ GILANI ET AL: "Development of respirable nanomicelle carriers for delivery of amphotericin B by jet nebulization", JOURNAL OF PHARMACEUTICAL SCIENCES, vol. 100, no. 1, 2 janvier 2011 (2011-01-02), pages 252-259, XP055122741, ISSN: 0022-3549, DOI: 10.1002/jps.22274**
- **ESMAEIL MOAZENI ET AL: "Preparation and evaluation of inhalable itraconazole chitosan based polymeric micelles", DARU JOURNAL OF PHARMACEUTICAL SCIENCES, BIOMED CENTRAL LTD, LONDON, UK, vol. 20, 85, 3 décembre 2012 (2012-12-03), pages 1-9, XP021138107, ISSN: 2008-2231, DOI: 10.1186/2008-2231-20-85**
- **SHENGFANG SONG ET AL: "Self-aggregated nanoparticles based on amphiphilic poly(lactic acid)-grafted-chitosan copolymer for ocular delivery of amphotericin B", INTERNATIONAL JOURNAL OF NANOMEDICINE, 1 septembre 2013 (2013-09-01), page 3715, XP055122747, ISSN: 1176-9114, DOI: 10.2147/IJN.S51186**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- SIEW ADELINE ET AL: "Enhanced oral absorption of hydrophobic and hydrophilic drugs using quaternary ammonium palmitoyl glycol chitosan nanoparticles", MOLECULAR PHARMACEUTICS, ACS PUBLICATIONS, USA, vol. 9, no. 1, 1 janvier 2012 (2012-01-01) , pages 14-28, XP009167275, ISSN: 1543-8392, DOI: 10.1021/MP200469A
- MANNILA J ET AL: "Cyclodextrins and chitosan derivatives in sublingual delivery of low solubility peptides: A study using cyclosporin A, alpha-cyclodextrin and quaternary chitosan N-betainate", INTERNATIONAL JOURNAL OF PHARMACEUTICS, ELSEVIER BV, NL, vol. 381, no. 1, 20 octobre 2009 (2009-10-20), pages 19-24, XP026642138, ISSN: 0378-5173, DOI: 10.1016/J.IJPHARM.2009.07.012 [extrait le 2009-07-24]

## Description

### DOMAINE DE L'INVENTION

Le domaine de l'invention est celui du chitosane hydrophobisé ayant des activités biologiques, en particulier des activités antiparasitaires et/ou antifongiques. Ainsi, la présente invention concerne une composition selon les revendications comprenant du chitosane hydrophobisé et pouvant être utilisée comme agent antiparasitaire et/ou antifongique ou dans le traitement d'infections parasitaires et/ou fongiques.

La présente invention concerne une composition selon les revendications comprenant un agent antifongique et du chitosane hydrophobisé. La composition de l'invention peut être utilisée comme agent antifongique ou dans le traitement d'infections fongiques. L'association de l'agent antifongique et du chitosane hydrophobisé permet d'augmenter l'efficacité de l'agent antifongique et de diminuer les doses administrées.

### ÉTAT DE LA TECHNIQUE

Les infections parasitaires et antifongiques sont largement répandues et peuvent avoir des conséquences graves, notamment chez les sujets immunodéprimés.

Les infections parasitaires sont causées par des métazoaires ou des protozoaires qui parasitent l'organisme, comme par exemple les *Leishmania,* les *Trichomonas* ou les *Trypanosomes.*

*Trichomonas vaginalis* est un parasite prostite responsable du trichomonas urogénital, une des maladies sexuellement transmissibles les plus répandues. Environ 180 millions de personnes sont infectées dans le monde par ce parasite ; cette maladie a des conséquences importantes tant sur le plan médical que social et économique.

Le traitement conventionnel du trichomonas urogénital consiste en l'utilisation de métronidazole (MTZ), un dérivé 5-nitroimidazole de l'azomycine, le traitement est administré par voie orale. Cependant, l'absorption systémique d'une telle molécule représente un risque d'allergie et favorise l'apparition du phénomène de résistance à cette molécule. Un des inconvénients du MTZ est aussi sa contre-indication chez la femme enceinte dans les premiers mois de grossesse, notamment à cause de son possible effet mutagène. Aucun traitement alternatif n'est aujourd'hui disponible et de nouvelles approches pour le traitement du trichomonas sont nécessaires.

La présente invention propose donc une nouvelle stratégie thérapeutique antiparasitaire basée sur l'utilisation d'une composition comprenant du chitosane hydrophobisé.

Le chitosane hydrophobisé de l'invention peut également être utilisé dans le traitement d'infections fongiques.

Les infections fongiques invasives sont causées par des organismes fongiques de type levures ou filaments. Les champignons responsables sont la plupart du temps inoffensifs. Cependant, lorsqu'ils infectent une personne immunodéprimée atteinte, par exemple, de cancer, du VIH (virus de l'immunodéficience humaine), ou traitée par des immunosuppresseurs, ces mêmes champignons peuvent s'avérer très pathogènes. Les champignons, responsables d'infections chez l'homme, les plus souvent isolés sont *Candida sp.* et *Aspergillus sp.* Divers traitements existent pour traiter les infections fongiques mais l'augmentation de leur fréquence depuis une trentaine d'années, ainsi que l'apparition de résistances, rendent nécessaire la mise au point de nouveaux traitements.

Des molécules de plus en plus performantes comme les azolés de troisième génération, les polyènes ou les échinocandines, sont aujourd'hui utilisées pour traiter les infections fongiques. Les azolés, tels que l'itraconazole, le voriconazole ou le fluconazole, sont des antifongiques à large spectre qui sont efficaces mais responsables d'un risque accru d'interactions médicamenteuses et qui entraînent des troubles hépatiques. Les polyènes sont des molécules amphiphiles volumineuses, telles que la nystatine ou l'amphotéricine B, possédant également une activité à large spectre. Cependant, la nystatine est réservée à l'usage topique en raison d'une toxicité élevée. L'amphotéricine B, quant à elle, peut être administrée par voie orale mais présente une toxicité rénale. Les échinocandines visent à détruire la paroi cellulaire fongique en inhibant la β (1-3)-D-glucane synthétase. Ces antifongiques présentent un spectre d'action étendu et une grande efficacité mais leur utilisation représente un coût très élevé et ils sont responsables de l'apparition de souches résistantes.

Les antifongiques actuels présentent donc divers inconvénients, liés principalement à la nécessité d'utiliser des doses importantes et/ou répétées :

- existence d'effets secondaires ;
- apparition de résistances, ce qui peut notamment avoir des conséquences dramatiques chez les sujets immunodéprimés ;
- coût élevé des traitements.

Il existe donc un besoin pour de nouveaux traitements antifongiques permettant de réduire les doses administrées afin de limiter les effets secondaires, d'éviter l'apparition de résistances et de diminuer le coût des traitements.

Des formulations ont été proposées afin de vectoriser des agents antifongiques et favoriser leur disponibilité. La vectorisation permet notamment de véhiculer l'agent antifongique dans l'organisme sans qu'il ne soit altéré ni ne provoquer d'effets secondaires, puis de le libérer au niveau du tissu cible. En augmentant la disponibilité de l'agent antifongique, des doses moins importantes peuvent être administrées pour une même efficacité.

Ainsi, de l'amphotéricine B (AmB) encapsulée dans des liposomes (Ambisome) est aujourd'hui disponible commercialement. L'amphotéricine B existe également sous forme d'Abelcet, une formulation en bicouche lipidique. Des travaux ont par ailleurs décrit la préparation de nanoparticules lipidiques cationiques comme vecteurs de l'amphotéricine B (Vieira et al. J. Nanobiotech. 2008, 6 :6). Ces nanoparticules ont été testées contre le champignon *C. albicans* : elles ont montré une activité antifongique équivalente à celle de la Fungizone® (AmB dans du déoxycholate) tout en limitant la néphrotoxicité.

Diverses formulations comprenant du chitosane ont également été proposées. Le chitosane est un polysaccharide naturel qui présente l'avantage d'être biocompatible, biodégradable et bioadhésif. Ainsi, des liposomes d'AmB revêtus de chitosane ont été décrits pour améliorer la disponibilité pulmonaire de l'AmB (Albasarah et al., J. Pharmacy Pharmacol., 2010, 62, 821-828). Des particules de chitosane ont pour leur part été utilisées pour encapsuler de l'AmB et ont montré une efficacité antifongique équivalente à celle de la Fungizone®, tout en présentant une toxicité rénale réduite (Limpeanchob et al., Naresuan Univ. J., 2006, 14(2) 27-34).

Les formulations ci-dessus permettent donc de diminuer les effets secondaires de l'amphotéricine B et/ou d'améliorer la biodisponibilité.

Une autre stratégie pour diminuer les doses d'antifongique administrées est de fournir des agents antifongiques présentant une activité biologique plus importante. Une efficacité accrue peut alors être obtenue pour des doses réduites.

Ainsi, Gharib et *al.* ont montré que des nanosphères de PLGA (poly[lactid-co-glycolid]) chargées avec de AmB permettaient de réduire la toxicité de l'AmB et d'augmenter son activité (Gharib et al. DARU, 2011, 19(5), 351-355). Toutefois, la préparation des nanosphères fait appel à la technique de nanoprécipitation. Cette technique présente l'inconvénient d'utiliser des solvants toxiques tels que l'acétone.

La présente invention concerne une composition comprenant un agent antifongique et du chitosane modifié (chitosane hydrophobisé), le chitosane modifié pouvant former des particules. Gilani et *al.,* ont décrit la vectorisation d'un agent antifongique par des micelles de chitosane hydrophobisé à partir d'un chitosane déacétylé à 95%, puis dépolymérisé. (Gilani et al., J. Pharm Sci, 2011, 100(1), 252-259). De plus, Moazeni et al., ont décrit l'emploi de l'itraconazole vectorisé à l'aide d'une composition miscellaire constituée d'un dérivé de chitosane initialement déacétylé à hauteur de 95% et hydrophobisé par réaction avec l'acide stéarique (Moazeni et al., Daru J. Pharm. Sc., 20, 85, 1-9).

A la connaissance de la Demanderesse, les particules constituées de chitosane hydrophobisé de l'invention en association avec une cyclodextrine, formant avec le chitosane hydrophobisé un complexe d'inclusion non-covalent, n'ont jamais été décrites dans l'art antérieur. La préparation des compositions de l'invention se fait par simple mélange des constituants. La Demanderesse a mis en évidence que la composition de l'invention présente une activité antifongique plus élevée que celle de l'agent antifongique seul et permet donc de diminuer les doses nécessaires pour traiter les infections fongiques.

## DÉFINITIONS

Dans la présente invention, les termes ci-dessous sont définis de la manière suivante :

- « **agent antifongique** » fait référence à une substance possédant la capacité de tuer ou de limiter la prolifération des champignons microscopiques et des levures.
- « **agent antiparasitaire** » fait référence à une substance possédant la capacité de tuer ou de limiter la prolifération des parasites.
- « **chitosane** » fait référence à un hétéropolymère linéaire de D-glucosamine et de *N*-acétyle-D-glucosamine liés en b (1-4) selon la formule suivante :

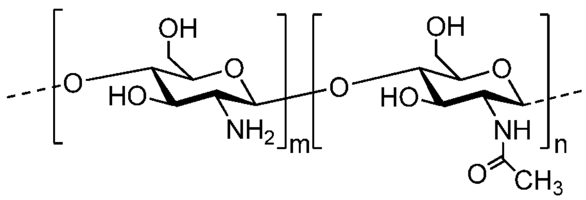

dans laquelle m représente le nombre d'unités D-glucosamine et n représente le nombre d'unités *N*-acétyle-D-glucosamine ; sous réserve que le pourcentage de m par rapport au nombre total d'unité soit supérieur à 50%.

- « **chitosane hydrophobisé** » signifie que le chitosane a été rendu hydrophobe par greffage sur les fonctions hydroxyles et/ou amines, de chaînes qui, par nature, sont hydrophobes en raison de leur caractère apolaire, de préférence des chaines alkyles ou alcényles. Selon un mode de réalisation préféré, le chitosane hydrophobisé de l'invention est amphiphile.

- « **cyclodextrine** » fait référence à un oligosaccharide cyclique d'unités β-D-glucopyranoses reliées par des liaisons α(1-4). Les cyclodextrines sont des molécules-cages. Il en existe de différentes tailles, ayant toutes la forme d'un abat-jour et portant des groupes hydrophiles localisés sur l'extérieur, la cavité étant relativement hydrophobe.

La cyclodextrine utilisée dans l'invention est « **une α-cyclodextrine** ». Cette expression fait référence à l'a-cyclodextrine ou un dérivé de l'a-cyclodextrine, l'a-cyclodextrine étant formée de 6 sous-unités glucopyranose. L'a-cyclodextrine est disponible dans le commerce et est reconnue comme étant un excipient pharmaceutiquement accepté par la majorité des pharmacopées.

- « **complexe d'inclusion** » désigne un système qui résulte de l'interaction entre une molécule « hôte » qui admet à l'intérieur de sa cavité une ou plusieurs autres molécules « invitées », sans qu'aucune liaison covalente ne s'établisse. L'expression « **complexe d'inclusion non-covalent** » signifie que les constituants du complexe d'inclusion interagissent par des liaisons de Van der Waals et/ou des liaisons hydrogène, et/ou des liaisons électrostatiques, et/ou des liaisons hydrophobes, à l'exclusion des liaisons covalentes.
- « **groupe hydrophobe** » fait référence à un substituant apolaire, qui ne peut engager de liaisons hydrogènes et qui se dissous plus facilement dans l'huile ou d'autres solvants hydrophobes que dans l'eau.
- « **alkyle** » fait référence à une chaîne hydrocarbonée linéaire ou branché, saturée, comportant de préférence de 1 à 20 atomes de carbone, de préférence méthyle, éthyle, propyle, isopropyle, n-butyle, sec-butyle, isobutyle, tert-butyle, lauryl -C, ou des chaines alkyle comprenant 11, 13, 15 ou 17 atomes de carbones.
- « **alcényle** » fait référence à toute chaîne hydrocarbonée linéaire ou ramifiée, portant au moins une double liaison, de préférence 1, 2, 3 ou 4 doubles liaisons, de 2 à 20 atomes de carbone. Dans un mode de réalisation particulier de l'invention, « alcényle » fait référence à une chaîne hydrocarbonée linéaire, portant 1, 2 ou 3 doubles liaisons, et comprenant 11, 13, 15 ou 17 atomes de carbones.
- « **poly(alkylcyanoacrylate)** » fait référence à un polymère obtenu par polymérisation d'unités alkylcyanoacrylates.
- « **pharmaceutiquement acceptable** » fait référence à un composé qui ne produit pas une réaction allergique ou réaction indésirable lorsqu'il est administré à un animal, de préférence un humain. Les excipients pharmaceutiquement acceptables comprennent tous les solvants, les milieux de dispersion, les revêtements, les agents antibactériens et antifongiques, les agents isotoniques, les agents retardant l'absorption et autres agents similaires.
- « **patient** » fait référence à un animal à sang chaud, de préférence un être humain, qui est en attente de la réception de, ou reçoit des soins médicaux ou est/sera l'objet d'une procédure médicale ou est suivi pour le développement d'une maladie.

## DESCRIPTION DÉTAILLÉE

### Composition

La présente invention concerne une composition selon les revendications comprenant du chitosane hydrophobisé.

La composition comprend :

➢ du chitosane hydrophobisé de formule générale (I) :

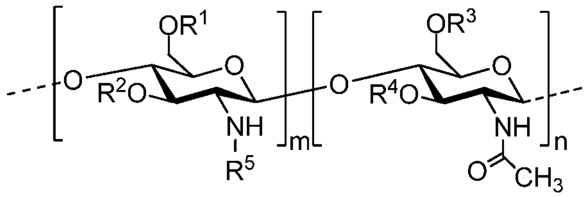

dans laquelle

- $R^1$, $R^2$, $R^3$, $R^4$ et $R^5$ sont identiques ou différents, et représentent :
  - un atome d'hydrogène ;
  - un groupe hydrophobe ;
  - un groupe substitué par une fonction thiol ;

  sous réserve qu'au moins l'un de $R^1$, $R^2$, $R^3$, $R^4$ et $R^5$ est un groupe hydrophobe ;
- n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50%.

*Chitosane hydrophobisé*

Selon un mode de réalisation, le chitosane hydrophobisé est composé d'au moins trois unités saccharidiques, de préférence de 5 à 1500 unités saccharidiques, plus préférentiellement de 25 à 100 unités saccharidiques. Dans un mode de réalisation, le chitosane hydrophobisé de formule (I) est tel que m+n est au moins égal à 3, de préférence m+n va de 3 à 6000, plus préférentiellement de 5 à 1500. Puisqu'il s'agit de chitosane, le pourcentage de m par rapport à m+n (degré d'acétylation) doit être supérieur à 50%. Un degré d'acétylation inférieur à 50% correspond à de la chitine.

Selon un mode de réalisation, la masse molaire du chitosane hydrophobisé est comprise entre 5 kDa et 100 000 kDa, de préférence entre 50 kDa et 10 000 kDa, plus préférentiellement entre 100 kDa et 1000 kDa. Selon un mode de réalisation préféré, la masse molaire du chitosane hydrophobisé est égale à 10 kDa, 20 kDa, 145 kDa ou 250 kDa.

Selon un mode de réalisation de la présente invention, le chitosane hydrophobisé présente un degré de substitution par les groupements hydrophobes allant de 0,001 à 100%, de préférence de 0,05 à 70%, plus préférentiellement de 0,1 à 50%, plus préférentiellement de 1 à 20%. Le degré de substitution reflète le nombre de groupes hydrophobes liés à 100 unités saccharidiques de la chaine polysaccharidique du chitosane. Il peut être déterminé par les conditions expérimentales du greffage et peut être mesuré par RMN ou par analyse élémentaire par exemple.

Selon un mode de réalisation, les groupements hydrophobes sont liés de façon covalente au chitosane par un ou plusieurs atomes d'azote dudit chitosane. Dans un autre mode de réalisation, les groupements hydrophobes sont liés de façon covalente au chitosane par un ou plusieurs atomes d'oxygène dudit chitosane. Dans un autre mode de réalisation les groupements hydrophobes sont liés de façon covalente au chitosane par un ou plusieurs atomes d'azote et/ou un ou plusieurs atomes d'oxygène dudit chitosane.

Selon un mode de réalisation, le chitosane hydrophobisé utilisé dans la composition de l'invention est obtenu par des méthodes de synthèse connues de l'homme du métier. En particulier, la fonctionnalisation du chitosane sur un ou plusieurs de ses atomes d'azote par des chaines hydrophobes peut être réalisée par *N*-acylation, en présence d'un agent de couplage et d'un acide gras. La fonctionnalisation du chitosane sur un ou plusieurs de ses atomes d'oxygène par des chaines hydrophobes peut être réalisée par *O*-acylation, en présence d'un acide gras. Des méthodes de synthèse du chitosane hydrophobisé selon l'invention sont présentées dans la partie expérimentale.

Selon un mode de réalisation, le chitosane hydrophobisé de formule générale (I) est tel que le groupe hydrophobe est sélectionné parmi :

◦ un groupe de formule $-COR^6$, dans lequel $R^6$ représente :

  ▪ un groupe alkyle linéaire ou ramifié, comprenant de 1 à 1000 atomes de carbone, de préférence de 1 à 20 atomes de carbone, plus préférentiellement les groupes $-(CH_2)_{14}-CH_3$ ou $-(CH_2)_{16}-CH_3$ ;

  ▪ un groupe alcényle linéaire ou ramifié, comprenant de 2 à 1000 atomes de carbone, de préférence de 2 à 20 atomes de carbone, et comprenant au moins une double liaison C=C, de préférence de 1 à 4 doubles liaisons, de préférence les groupes $-(CH_2)_7-CH=CH-CH_2-(CH_2)_7-CH_3$ ou $-(CH_2)_7-CH=CH-(CH_2)_7-CH_3$ ;

◦ un poly(alkylcyanoacrylate) dans lequel l'alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate).

Selon un mode de réalisation, le chitosane hydrophobisé de formule générale (I) est tel que le groupe substitué par une fonction thiol est un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant

de 1 à 10, de préférence 1, 2, 3, 4, ou 5.

Selon un mode de réalisation, le chitosane hydrophobisé est de formule générale (I) :

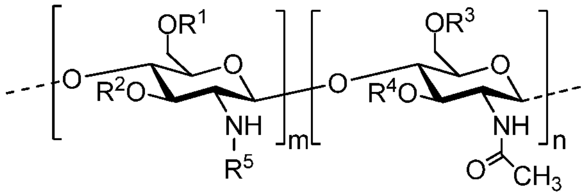

dans laquelle

- $R^1$, $R^2$, $R^3$, $R^4$ et $R^5$ sont identiques ou différents, et représentent :
  - un atome d'hydrogène ;
  - un groupe hydrophobe sélectionné parmi :
    - un groupe de formule $-COR^6$, dans lequel $R^6$ représente :
      - un groupe alkyle linéaire ou ramifié, comprenant de 1 à 1000 atomes de carbone, de préférence de 1 à 20 atomes de carbone, plus préférentiellement les groupes $-(CH_2)_{14}-CH_3$ ou $-(CH_2)_{16}-CH_3$ ;
      - un groupe alcényle linéaire ou ramifié, comprenant de 2 à 1000 atomes de carbone, de préférence de 2 à 20 atomes de carbone, et comprenant au moins une double liaison C=C, de préférence de 1 à 4 doubles liaisons, de préférence les groupes $-(CH_2)_7-CH=CH-CH_2-(CH_2)_7-CH_3$ ou $-(CH_2)_7-CH=CH-(CH_2)_7-CH_3$ ;
    - un poly(alkylcyanoacrylate) dans lequel l' alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate) ;
  - un groupe substitué par une fonction thiol, de préférence un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant de 1 à 10, de préférence 1, 2, 4, ou 5 ;
  sous réserve que au moins l'un de $R^1$, $R^2$, $R^3$, $R^4$ et $R^5$ est un groupe hydrophobe ;
- n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50%.

Selon un mode de réalisation, le chitosane hydrophobisé est de formule générale (Ia) :

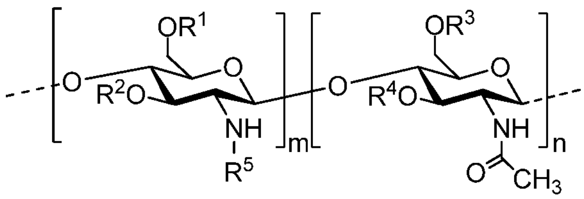

dans laquelle

- $R^1$, $R^2$, $R^3$, $R^4$ et $R^5$ sont identiques ou différents, et représentent :
  - un atome d'hydrogène ;
  - un groupe hydrophobe de formule $-COR^6$, dans lequel $R^6$ représente :

- un groupe alkyle linéaire ou ramifié, comprenant de 1 à 1000 atomes de carbone, de préférence de 1 à 20 atomes de carbone, plus préférentiellement les groupes $-(CH_2)_{14}-CH_3$ ou $-(CH_2)_{16}-CH_3$ ;
- un groupe alcényle linéaire ou ramifié, comprenant de 2 à 1000 atomes de carbone, de préférence de 2 à 20 atomes de carbone, et comprenant au moins une double liaison C=C, de préférence de 1 à 4 doubles liaisons, de préférence les groupes $-(CH_2)_7-CH=CH-CH_2-(CH_2)_7-CH_3$ ou $-(CH_2)_7-CH=CH-(CH_2)_7-CH_3$ ;

- un groupe substitué par une fonction thiol, de préférence un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant de 1 à 10, de préférence 1, 2, 4, ou 5 ;

sous réserve qu'au moins l'un de $R^1$, $R^2$, $R^3$, $R^4$ et $R^5$ est un groupe hydrophobe ;

- n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50%.

Selon un mode de réalisation particulier, les groupements hydrophobes portés par le chitosane hydrophobisé de formule (la) sont obtenus par couplage sur les fonctions amines et/ou hydroxyles du chitosane de l'acide laurique, l'acide palmitique, l'acide oléique, l'acide stéarique, l'acide linoléique, cette liste n'étant en aucun cas limitative.

Selon un mode de réalisation, le chitosane hydrophobisé est de formule générale (Ib) :

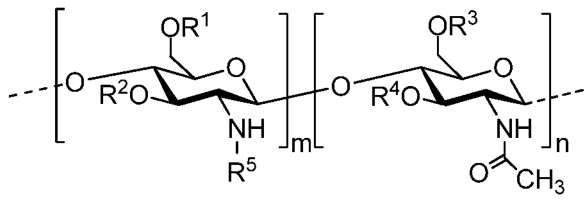

dans laquelle

- $R^1$, $R^2$, $R^3$, $R^4$ et $R^5$ sont identiques ou différents, et représentent :
  - un atome d'hydrogène ;
  - un groupe hydrophobe poly(alkylcyanoacrylate) dans lequel l'alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate) ;
  - un groupe substitué par une fonction thiol, de préférence un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant de 1 à 10, de préférence 1, 2, 4, ou 5 ;

  sous réserve qu'au moins l'un de $R^1$, $R^2$, $R^3$, $R^4$ et $R^5$ est un groupe hydrophobe ;
- n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50%.

Selon un mode de réalisation particulier, le chitosane hydrophobisé est de formule générale (Ib) dans laquelle $R^5$ représente un groupe hydrophobe poly(alkylcyanoacrylate) dans lequel l'alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate).

Selon un mode de réalisation particulier, le chitosane hydrophobisé est de formule générale (Ib) dans laquelle $R^5$ représente un groupe hydrophobe poly(alkylcyanoacrylate) tel que défini ci-dessus et au moins l'un de $R^1$, $R^2$, $R^3$ et $R^4$ représentent un groupe substitué par une fonction thiol.

Selon un autre mode de réalisation particulier, le chitosane hydrophobisé est de formule générale (Ib) dans laquelle $R^5$ représente un groupe hydrophobe poly(alkylcyanoacrylate) tel que défini ci-dessus et $R^1$, $R^2$, $R^3$ et $R^4$ représentent H.

Selon un mode de réalisation préféré, la composition de l'invention comprend du chitosane hydrophobisé de

formule générale (Ib) telle que décrite ci-dessus.

Selon un mode de réalisation, le chitosane hydrophobisé de formule générale (Ib) est présent sous forme de particules dans la composition de l'invention, de préférence sous forme de nanoparticules ou de microparticules.

Selon un mode de réalisation, la taille des nanoparticules de chitosane hydrophobisé de formule générale (Ib) est comprise entre 10 et 200000 nm, de préférence entre 50 et 100000 nm, plus préférentiellement entre 100 et 80000 nm.

Selon un mode de réalisation, les particules de chitosane hydrophobisé de formule générale (Ib) comprenant des chaines poly(alkylcyanoacrylate) sont obtenues par polymérisation anionique ou radicalaire, de préférence en émulsion, à partir de chitosane et de monomère alkylcyanoacrylate.

Selon un mode de réalisation particulier, la composition de l'invention comprend :

(i) du chitosane hydrophobisé de formule générale (Ib) dans laquelle $R^5$ représente un groupe hydrophobe poly(alkylcyanoacrylate) tel que défini ci-dessus et au moins l'un de $R^1$, $R^2$, $R^3$ et $R^4$ représentent un groupe substitué par une fonction thiol ;
et
(ii) du chitosane hydrophobisé de formule générale (Ib) dans laquelle $R^5$ représente un groupe hydrophobe poly(alkylcyanoacrylate) tel que défini ci-dessus et $R^1$, $R^2$, $R^3$ et $R^4$ représentent H.

Selon un mode de réalisation particulier, la composition de l'invention comprend de 0% à 100% en masse de chitosane hydrophobisé (i) par rapport à la masse totale de chitosane hydrophobisé présent dans la composition, de préférence de 25 à 75%.

Selon un mode de réalisation particulier, la composition de l'invention comprend 25% en masse de chitosane hydrophobisé (i) et 75% en masse de chitosane hydrophobisé (ii), par rapport à la masse totale de chitosane hydrophobisé présent dans la composition.

Selon un mode de réalisation particulier, la composition de l'invention comprend 50% en masse de chitosane hydrophobisé (i) et 50% en masse de chitosane hydrophobisé (ii), par rapport à la masse totale de chitosane hydrophobisé présent dans la composition.

Selon un mode de réalisation particulier, la composition de l'invention comprend 75% en masse de chitosane hydrophobisé (i) et 25% en masse de chitosane hydrophobisé (ii), par rapport à la masse totale de chitosane hydrophobisé présent dans la composition.

*Chitosane hydrophobisé + cyclodextrine*

La composition de l'invention comprend en outre une $\alpha$-cyclodextrine.

Le chitosane hydrophobisé forme avec l'a-cyclodextrine un complexe d'inclusion non-covalent. Le complexe d'inclusion non-covalent peut former des particules.

La composition de l'invention comprend une $\alpha$-cyclodextrine présente sous forme de monomère et formant avec le chitosane hydrophobisé de formule générale (I) un complexe d'inclusion non-covalent.

L'$\alpha$-cyclodextrine peut être substituée ou non-substituée. Par cyclodextrine substituée, on entend par exemple une cyclodextrine substituée par un groupe alkyle, par un groupe hydroxyalkyle, par un groupe maltosyl ou par un groupe galactosyl.

Selon un mode de réalisation, l'$\alpha$-cyclodextrine de la composition de l'invention est de formule générale (II) :

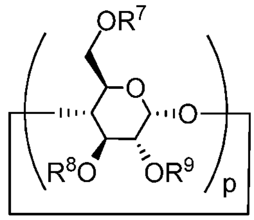

dans laquelle

- p est égal à 6,
- $R^7$, $R^8$ et $R^9$ sont identiques ou différents, de préférence identiques, et représentent chacun indépendamment un atome d'hydrogène ; un groupement alkyle comportant 1 à 4 atomes de carbone, de préférence choisi dans le groupe comprenant méthyle, éthyle, propyle, et isopropyle ; $-NH_2$, $-NH_3^+$, ou $-SO_4^{2-}$ ; de préférence, $R^7$, $R^8$ et $R^9$ représentent chacun indépendamment un atome d'hydrogène ou un groupement méthyle.

Selon un mode de réalisation particulier, l' α-cyclodextrine se présente sous la forme d'une α-cyclodextrine fonctionnalisée par un ligand choisi parmi des anticorps, des fragments d'anticorps, des récepteurs, des lectines, de la biotine ou ses dérivés.

Dans un mode de réalisation de l'invention, la composition comprend :

- du chitosane hydrophobisé de formule générale (I) :

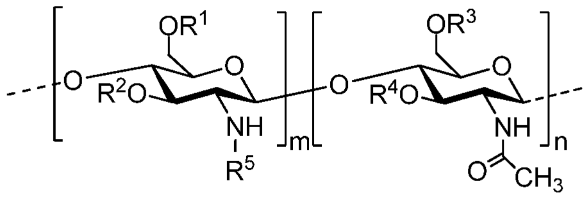

dans laquelle

- $R^1$, $R^2$, $R^3$, $R^4$ et $R^5$ sont identiques ou différents, et représentent :
  - un atome d'hydrogène ;
  - un groupe hydrophobe sélectionné parmi :
    - un groupe de formule $-COR^6$, dans lequel $R^6$ représente :
      - un groupe alkyle linéaire ou ramifié, comprenant de 1 à 1000 atomes de carbone, de préférence de 1 à 20 atomes de carbone, plus préférentiellement les groupes $-(CH_2)_{14}-CH_3$ ou $-(CH_2)_{16}-CH_3$ ;
      - un groupe alcényle linéaire ou ramifié, comprenant de 2 à 1000 atomes de carbone, de préférence de 2 à 20 atomes de carbone, et comprenant au moins une double liaison C=C, de préférence de 1 à 4 doubles liaisons, de préférence les groupes $-(CH_2)_7-CH=CH-CH_2-(CH_2)_7-CH_3$ ou $-(CH_2)_7-CH=CH-(CH_2)_7-CH_3$ ;
    - un poly(alkylcyanoacrylate) dans lequel l' alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate) ;
  - un groupe substitué par une fonction thiol, de préférence un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant de 1 à 10, de préférence 1, 2, 3, 4, ou 5 ;

  sous réserve qu'au moins l'un de $R^1$, $R^2$, $R^3$, $R^4$ et $R^5$ est un groupe hydrophobe ;
- n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50% ;

- une α-cyclodextrine, ladite α-cyclodextrine étant sous forme de monomère, et formant avec le chitosane hydrophobisé de formule générale (I) un complexe d'inclusion non-covalent.

Selon un aspect de l'invention, la composition de l'invention comprend :

- du chitosane hydrophobisé de formule générale (Ia) telle que décrite ci-dessus ;
- une α-cyclodextrine, ladite α-cyclodextrine étant sous forme de monomère, et formant avec le chitosane hydrophobisé de formule générale (Ia) un complexe d'inclusion non-covalent.

Dans cet aspect de l'invention, selon un mode de réalisation préféré, l'a-cyclodextrine est de formule générale (II), telle que décrite ci-dessus.

Selon un mode de réalisation, le chitosane hydrophobisé de formule générale (Ia) forme avec l'a-cyclodextrine un complexe d'inclusion non-covalent. Les complexes d'inclusion forment des particules, de préférence des nanoparticules ou des microparticules.

Dans un mode de réalisation, la taille des particules de complexe d'inclusion est comprise entre 10 et 200 000 nm, de préférence entre 50 et 100 000 nm. Selon un mode de réalisation, les particules de complexe d'inclusion sont des nanoparticules dont la taille est comprise entre 10 et 1000 nm, de préférence entre 50 et 1000 nm. Selon un mode de réalisation, les particules de complexe d'inclusion sont des microparticules dont la taille est comprise entre 1000 et 50000 nm, de préférence entre 1000 et 10000 nm.

Avantageusement, le rapport entre la concentration d'$\alpha$-cyclodextrine et la concentration du chitosane hydrophobisé de formule générale (Ia) a une valeur allant de 0,01 à 1500, de préférence de 4 à 15, plus préférentiellement égal à 10. En modifiant ce rapport de concentration, il est possible de moduler la taille des particules obtenue à partir des complexes d'inclusion.

Selon un mode de réalisation, dans la particule de complexe d'inclusion de chitosane hydrophobisé de formule générale (Ia) et d'$\alpha$-cyclodextrine, de préférence d'$\alpha$-cyclodextrine de formule (II), le chitosane hydrophobisé de formule générale (Ia) est présent à une concentration allant de 0,1 à 30 % en masse par rapport à la masse totale de la particule, de préférence de 0,5 à 20%, plus préférentiellement de 0,5 à 10 %.

Selon un mode de réalisation, dans la particule de complexe d'inclusion de chitosane hydrophobisé de formule générale (Ia) et d'$\alpha$-cyclodextrine, de préférence d'$\alpha$-cyclodextrine de formule (II), l'$\alpha$-cyclodextrine est présente à une concentration allant de 0,1 à 99% en masse par rapport à la masse totale de la particule, de préférence de 4 à 25%, plus préférentiellement égal à 10%.

*Chitosane hydrophobisé + agent antifongique*

La composition de l'invention comprend en outre un agent antifongique.

La présente invention concerne une composition comprenant un agent antifongique et du chitosane hydrophobisé.

Dans un mode de réalisation, le chitosane hydrophobisé est présent dans la composition de l'invention sous forme de particules. Dans un premier mode de réalisation, l'agent antifongique et les particules de chitosane hydrophobisé sont en association dans la composition de l'invention, c'est à dire que l'agent antifongique n'est pas encapsulé, il est simplement mélangé avec les particules chitosane hydrophobisé. Dans un second mode de réalisation, l'agent antifongique est encapsulé par les particules de chitosane hydrophobisé, c'est-à-dire qu'une partie de l'agent antifongique se trouve « piégée » à l'intérieur de la particule et une partie se trouve à l'extérieur de la particule.

La composition de l'invention comprend en outre une $\alpha$-cyclodextrine. Dans un mode de réalisation, le chitosane hydrophobisé forme avec l'a-cyclodextrine un complexe d'inclusion non-covalent. Le complexe d'inclusion non-covalent peut former des particules. Dans un premier mode de réalisation, l'agent antifongique et le complexe d'inclusion de chitosane hydrophobisé et de cyclodextrine sont en association dans la composition de l'invention. Dans un second mode de réalisation, l'agent antifongique est encapsulé par le complexe d'inclusion de chitosane hydrophobisé et de cyclodextrine.

Dans un mode de réalisation de l'invention, la composition comprend :

- au moins un agent antifongique ;
- du chitosane hydrophobisé de formule générale (I) :

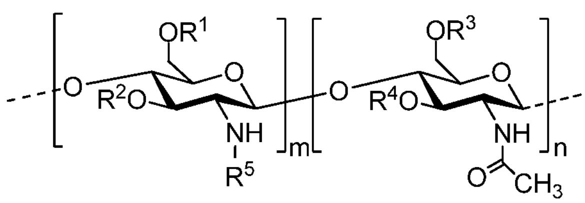

dans laquelle

- $R^1$, $R^2$, $R^3$, $R^4$ et $R^5$ sont identiques ou différents, et représentent :
  - un atome d'hydrogène ;
  - un groupe hydrophobe ;

- un groupe substitué par une fonction thiol ;

sous réserve qu'au moins l'un de $R^1$, $R^2$, $R^3$, $R^4$ et $R^5$ est un groupe hydrophobe ;

- n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50%.

Selon un mode de réalisation, le chitosane hydrophobisé de formule générale (I) est tel que le groupe hydrophobe est sélectionné parmi :

◦ un groupe de formule $-COR^6$, dans lequel $R^6$ représente :

▪ un groupe alkyle linéaire ou ramifié, comprenant de 1 à 1000 atomes de carbone, de préférence de 1 à 20 atomes de carbone, plus préférentiellement les groupes $-(CH_2)_{14}-CH_3$ ou $-(CH_2)_{16}-CH_3$ ;
▪ un groupe alcényle linéaire ou ramifié, comprenant de 2 à 1000 atomes de carbone, de préférence de 2 à 20 atomes de carbone, et comprenant au moins une double liaison C=C, de préférence de 1 à 4 doubles liaisons, de préférence les groupes $-(CH_2)_7-CH=CH-CH_2-(CH_2)_7-CH_3$ ou $-(CH_2)_7-CH=CH-(CH_2)_7-CH_3$ ;

◦ un poly(alkylcyanoacrylate) dans lequel l'alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate).

Selon un mode de réalisation, le chitosane hydrophobisé de formule générale (I) est tel que le groupe substitué par une fonction thiol est un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant de 1 à 10, de préférence 1, 2, 3, 4, ou 5.

La composition de l'invention comprend en outre une α-cyclodextrine, ladite α-cyclodextrine étant sous forme de monomère et formant avec le chitosane hydrophobisé de formule générale (I) un complexe d'inclusion non-covalent.

Dans un mode de réalisation de l'invention, la composition comprend :

➢ au moins un agent antifongique ;
➢ du chitosane hydrophobisé de formule générale (I) :

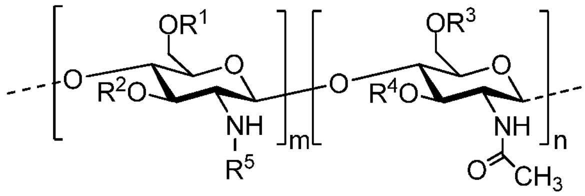

dans laquelle

- $R^1$, $R^2$, $R^3$, $R^4$ et $R^5$ sont identiques ou différents, et représentent :

  - un atome d'hydrogène ;
  - un groupe hydrophobe sélectionné parmi :

◦ un groupe de formule $-COR^6$, dans lequel $R^6$ représente :

▪ un groupe alkyle linéaire ou ramifié, comprenant de 1 à 1000 atomes de carbone, de préférence de 1 à 20 atomes de carbone, plus préférentiellement les groupes $-(CH_2)_{14}-CH_3$ ou $-(CH_2)_{16}-CH_3$ ;
▪ un groupe alcényle linéaire ou ramifié, comprenant de 2 à 1000 atomes de carbone, de préférence de 2 à 20 atomes de carbone, et comprenant au moins une double liaison C=C, de préférence de 1 à 4 doubles liaisons, de préférence les groupes $-(CH_2)_7-CH=CH-CH_2-(CH_2)_7-CH_3$ ou $-(CH_2)_7-CH=CH-(CH_2)_7-CH_3$ ;

◦ un poly(alkylcyanoacrylate) dans lequel l' alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate) ;

- un groupe substitué par une fonction thiol, de préférence un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant de 1 à 10, de préférence 1, 2, 3, 4, ou 5 ;

sous réserve que au moins l'un de $R^1$, $R^2$, $R^3$, $R^4$ et $R^5$ est un groupe hydrophobe ;

• n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50% ;

➢ une α-cyclodextrine, ladite α-cyclodextrine étant sous forme de monomère, et formant avec le chitosane hydrophobisé de formule générale (I) un complexe d'inclusion non-covalent.

Selon un mode de réalisation préféré, la composition de l'invention comprend :

➢ au moins un agent antifongique ; et

➢ du chitosane hydrophobisé de formule générale (Ib) telle que décrite ci-dessus.

Selon un mode de réalisation, le chitosane hydrophobisé de formule générale (Ib) est présent sous forme de particules dans la composition de l'invention, de préférence sous forme de nanoparticules ou de microparticules.

Selon un mode de réalisation, la taille des nanoparticules de chitosane hydrophobisé de formule générale (Ib) est comprise entre 10 et 200000 nm, de préférence entre 50 et 100000 nm, plus préférentiellement entre 100 et 80000 nm.

Selon un mode de réalisation, les particules de chitosane hydrophobisé de formule générale (Ib) comprenant des chaines poly(alkylcyanoacrylate) sont obtenues par polymérisation anionique ou radicalaire, de préférence en émulsion, à partir de chitosane et de monomère alkylcyanoacrylate.

Dans un premier mode de réalisation de l'invention, l'agent antifongique et les particules de chitosane hydrophobisé de formule générale (Ib) sont en association dans la composition de l'invention. Dans un second mode de réalisation, l'agent antifongique est encapsulé par les particules de chitosane hydrophobisé de formule générale (Ib).

Selon un mode de réalisation, la composition de l'invention comprend un agent antifongique choisi dans un groupe comprenant :

- les polyènes, tels que par exemple l'amphotéricine B, la candicidine, la filipine, l'hamycine, la natamycine, la nystatine, la rimocidine ;
- les composés imidazolés tels que par exemple le bifonazole, le butoconazole, le clotrimazole, l'éconazole, le fenticonazole, l'isoconazole, le kétoconazole, le miconazole, l'omoconazole, l'oxiconazole, le sertaconazole, le sulconazole, le tioconazole ;
- les composés triazolés, tels que par exemple l'albaconazole, le fluconazole, l'isavuconazole, l'itraconazole, le posaconazole, le ravuconazole, le terconazole, le voriconazole ;
- les composés thiazolés, tels que par exemple l'abafungine ;
- les allylamines, telles que par exemple l'amorolfine, la butenafine, la naftifine, la terbinafine;
- les échinocandines, telles que par exemple l'anidulafungine, la caspofungine, la micafungine ;
- l'acide benzoïque, la cérulénine, le ciclopirox, l'olamine, la flucytosine, la 5-fluorocytosine, la griséofulvine, l'haloprogine, le polygodial, le tolnaftate, l'acide undecylénique, le crystal violet ; ou
- la chlorhexidine, la polyvinylpyrrolidone (PVP) iodée (Bétadine®), le chlorure de benzalkonium (ovules ou crème vaginale Pharmatex®), le chlore (Dakin).

Selon un mode de réalisation préféré, l'agent antifongique est l'amphotéricine B ou la chlorhexidine, de préférence l'amphotéricine B.

*Chitosane hydrophobisé + cyclodextrine + agent antifongique*

Selon un premier aspect, la composition de l'invention comprend :

- au moins un agent antifongique ;
- du chitosane hydrophobisé de formule générale (la) telle que décrite ci-dessus ;
- une α-cyclodextrine, ladite α-cyclodextrine étant sous forme de monomère, et formant avec le chitosane hydrophobisé de formule générale (la) un complexe d'inclusion non-covalent.

Selon un mode de réalisation préféré, l'a-cyclodextrine est de formule générale (II), telle que décrite ci-dessus.

Selon un mode de réalisation, le chitosane hydrophobisé de formule générale (la) forme avec l'a-cyclodextrine un complexe d'inclusion non-covalent. Les complexes d'inclusion forment des particules, de préférence des nanoparticules ou des microparticules.

Dans un mode de réalisation, la taille des particules de complexe d'inclusion est comprise entre 10 et 200 000 nm, de préférence entre 50 et 100 000 nm. Selon un mode de réalisation, les particules de complexe d'inclusion sont des nanoparticules dont la taille est comprise entre 10 et 1000 nm, de préférence entre 50 et 1000 nm. Selon un mode de réalisation, les particules de complexe d'inclusion sont des microparticules dont la taille est comprise entre 1000 et 50000 nm, de préférence entre 1000 et 10000 nm.

Avantageusement, le rapport entre la concentration d'α-cyclodextrine et la concentration du chitosane hydrophobisé a une valeur allant de 0,01 à 1500, de préférence de 4 à 15, plus préférentiellement égal à 10. En modifiant ce rapport de concentration, il est possible de moduler la taille des particules obtenue à partir des complexes d'inclusion.

Selon un mode de réalisation, dans la particule de complexe d'inclusion de chitosane hydrophobisé de formule générale (la) et d'a-cyclodextrine, de préférence d'a-cyclodextrine de formule (II), le chitosane hydrophobisé de formule générale (la) est présent à une concentration allant de 0,1 à 30 % en masse par rapport à la masse totale de la particule, de préférence de 0,5 à 20%, plus préférentiellement de 0,5 à 10 %.

Selon un mode de réalisation, dans la particule de complexe d'inclusion de chitosane hydrophobisé de formule générale (la) et d'a-cyclodextrine, de préférence d'a-cyclodextrine de formule (II), l'α-cyclodextrine est présente à une concentration allant de 0,1 à 99% en masse par rapport à la masse totale de la particule, de préférence de 4 à 25%, plus préférentiellement égal à 10%.

Dans un premier mode de réalisation de l'invention, l'agent antifongique et les particules de complexe d'inclusion de chitosane hydrophobisé de formule (la) et de cyclodextrine sont en association dans la composition de l'invention. Dans un second mode de réalisation, l'agent antifongique est encapsulé par les particules de complexe d'inclusion de chitosane hydrophobisé de formule (la) et de cyclodextrine.

Selon un deuxième aspect, la composition de l'invention comprend :

- au moins un agent antifongique ;
- du chitosane hydrophobisé de formule générale (Ib) telle que décrite ci-dessus ;
- une α-cyclodextrine, ladite α-cyclodextrine étant sous forme de monomère, et formant avec le chitosane hydrophobisé de formule générale (Ib) un complexe d'inclusion non-covalent.

Selon un mode de réalisation préféré, la composition de l'invention comprend :

- au moins un agent antifongique ; et
- du chitosane hydrophobisé de formule générale (Ib) telle que décrite ci-dessus.

Selon un mode de réalisation, le chitosane hydrophobisé de formule générale (Ib) est présent sous forme de particules dans la composition de l'invention, de préférence sous forme de nanoparticules ou de microparticules.

Selon un mode de réalisation, la taille des nanoparticules de chitosane hydrophobisé de formule générale (Ib) est comprise entre 10 et 200000 nm, de préférence entre 50 et 100000 nm, plus préférentiellement entre 100 et 80000 nm.

Selon un mode de réalisation, les particules de chitosane hydrophobisé de formule générale (Ib) comprenant des chaines poly(alkylcyanoacrylate) sont obtenues par polymérisation anionique ou radicalaire, de préférence en émulsion, à partir de chitosane et de monomère alkylcyanoacrylate.

Dans un premier mode de réalisation de l'invention, l'agent antifongique et les particules de chitosane hydrophobisé de formule générale (Ib) sont en association dans la composition de l'invention. Dans un second mode de réalisation, l'agent antifongique est encapsulé par les particules de chitosane hydrophobisé de formule générale (Ib).

Selon un mode de réalisation, le chitosane hydrophobisé de formule générale (Ib) forme avec l'α-cyclodextrine un complexe d'inclusion non-covalent. Le complexe d'inclusion forme une particule, de préférence une nanoparticule ou une microparticule.

Dans un premier mode de réalisation de l'invention, l'agent antifongique et les particules de complexe d'inclusion, formées de particules de chitosane hydrophobisé de formule générale (Ib) et de cyclodextrine, sont en association dans la composition de l'invention. Dans un second mode de réalisation, l'agent antifongique est encapsulé par les particules de complexe d'inclusion de particules de chitosane hydrophobisé de formule générale (Ib) et de cyclodextrine.

**Procédé de fabrication de la composition de l'invention**

La présente invention concerne également un procédé de fabrication de la composition selon l'invention.

Selon un premier aspect, le procédé de fabrication de la composition selon l'invention comprend une étape de formation de particules de chitosane hydrophobisé : du chitosane hydrophobisé de formule générale (I) tel que décrit ci-dessus est placé dans un solvant, de préférence l'eau, en présence d'une $\alpha$-cyclodextrine, sous agitation, pour former une suspension de particules.

Selon un mode de réalisation particulier, un mélange de différents chitosanes hydrophobisés de formule générale (I) peut être utilisé pour préparer les particules de chitosane hydrophobisé de la composition de l'invention.

Avantageusement, les particules de chitosane hydrophobisé formées par le procédé de l'invention sont des nanoparticules ou des microparticules. Lorsqu'une $\alpha$-cyclodextrine est présente, il y a préférentiellement formation de complexes d'inclusion non-covalents entre le chitosane hydrophobisé et l'a-cyclodextrine. Les complexes d'inclusion peuvent former des particules, de préférence des nanoparticules ou des microparticules.

Selon un mode de réalisation particulier de l'invention, lors de l'étape de formation des particules, le chitosane hydrophobisé est solubilisé à une concentration allant de 0,01 à 9000 g/L, de préférence allant de 1 à 600 g/L, et notamment environ égale à 10 g/L, le solvant aqueux étant choisi parmi l'eau pure, une solution aqueuse de pH allant de 1 à 7 ou de 7 à 14, de préférence de 5 à 7, ou une solution de sérum physiologique optionnellement enrichie en glucose ou contenant tout autre excipient à usage pharmaceutique, médical, paramédical, dispositifs médicaux, alimentation animale, cosmétique, vétérinaire, agro-alimentaire, des pesticides, des cosméto-textiles, de la parfumerie, environnemental ou dans l'industrie des peintures, des emballages, du bâtiment et/ou de l'automobile.

Alternativement, lors de l'étape de formation des particules, le chitosane hydrophobisé est dispersé dans un milieu aqueux choisi parmi l'eau pure, une solution aqueuse de pH allant de 1 à 7 ou de 7 à 14, de préférence de 5 à 7, ou une solution de sérum physiologique optionnellement enrichie en glucose ou contenant tout autre excipient à usage pharmaceutique, médical, paramédical, dispositifs médicaux, alimentation animale, cosmétique, vétérinaire, agro-alimentaire, des pesticides, des cosméto-textiles, de la parfumerie, environnemental (dépollution des eaux par exemple) ou dans l'industrie des peintures, des emballages, du bâtiment et/ou de l'automobile.

La composition de l'invention comprend un agent antifongique. Le procédé de fabrication de la composition selon l'invention peut être un procédé d'association ou un procédé d'encapsulation.

Selon un premier aspect, l'invention concerne un procédé d'association, pour la préparation d'une composition selon l'invention.

Dans un mode de réalisation, le procédé d'association pour la préparation d'une composition selon l'invention, comprend les étapes suivantes :

(a) du chitosane hydrophobisé de formule générale (I) tel que décrit ci-dessus, est placé dans un solvant, de préférence l'eau, en présence d'une $\alpha$-cyclodextrine, sous agitation, pour former une suspension de particules ;
(b) au moins un antifongique est ajouté dans la suspension.

Avantageusement, lors de l'étape (a), lorsque le chitosane hydrophobisé est placé dans le solvant, il y a formation de particules, de préférence de nanoparticules ou de microparticules. Lorsqu'une $\alpha$-cyclodextrine est présente, il y a préférentiellement formation de complexes d'inclusion non-covalents entre le chitosane hydrophobisé et l'$\alpha$-cyclodextrine. Les complexes d'inclusion peuvent former des particules, de préférence des nanoparticules ou des microparticules.

Selon un mode de réalisation particulier de l'invention, lors de l'étape (a), le chitosane hydrophobisé est :

- en solution aqueuse à une concentration allant de 0,01 à 9000 g/L, de préférence allant de 1 à 600 g/L, et notamment environ égale à 10 g/L, le solvant aqueux étant choisi parmi l'eau pure, une solution aqueuse de pH allant de 1 à 7 ou de 7 à 14, de préférence de 5 à 7, ou une solution de sérum physiologique optionnellement enrichie en glucose ou contenant tout autre excipient à usage pharmaceutique, médical, paramédical, dispositifs médicaux, alimentation animale, cosmétique, vétérinaire, agro-alimentaire, des pesticides, des cosméto-textiles, de la parfumerie, environnemental ou dans l'industrie des peintures, des emballages, du bâtiment et/ou de l'automobile ;
- ou en dispersion dans un milieu aqueux choisi parmi l'eau pure, une solution aqueuse de pH allant de 1 à 7 ou de 7 à 14, de préférence de 5 à 7, ou une solution de sérum physiologique optionnellement enrichie en glucose ou contenant tout autre excipient à usage pharmaceutique, médical, paramédical, dispositifs médicaux, alimentation

animale, cosmétique, vétérinaire, agro-alimentaire, des pesticides, des cosméto-textiles, de la parfumerie, environnemental (dépollution des eaux par exemple) ou dans l'industrie des peintures, des emballages, du bâtiment et/ou de l'automobile.

Selon un second aspect, l'invention concerne un procédé d'encapsulation, pour la préparation d'une composition selon l'invention.

Dans un mode de réalisation, le procédé d'encapsulation pour la préparation d'une composition selon l'invention, comprend les étapes suivantes :

(a) au moins un agent antifongique est dissous dans un solvant, de préférence l'eau ;
(b) du chitosane hydrophobisé de formule générale (I), tel que décrit ci-dessus, et UNE $\alpha$-cyclodextrine, sont ajoutés dans la solution ;
(c) le mélange est agité pour former une suspension de particules de chitosane et de cyclodextrine, encapsulant l'agent antifongique.

Avantageusement, tout ou partie de l'agent antifongique se trouve alors encapsulé dans des particules de chitosane hydrophobisé et d'$\alpha$-cyclodextrine. En particulier, l'agent antifongique peut être encapsulé dans des particules comprenant des complexes d'inclusion non-covalents formés entre le chitosane hydrophobisé et l'a-cyclodextrine.

**Applications**

La présente invention concerne également une composition pharmaceutique, dermatologique, dermo-cosmétique ou vétérinaire comprenant la composition selon l'invention, en association avec un excipient pharmaceutiquement, dermatologiquement, dermo-cosmétiquement ou vétérinairement acceptable.

Des exemples d'excipients convenant pour ces modes de réalisation comprennent des solvants, des milieux de dispersion, agents isotoniques, agents retardant l'absorption, agents promoteurs d'absorption, lipides, protéines, peptides, sucres tels que le glucose ou le saccharose, tensioactifs, agents gélifiants, agents épaississants, agents liants, diluants, conservateurs, antioxydants, colorants, solubilisants, délitants, édulcorants.

Selon un mode de réalisation, la composition de l'invention, la composition pharmaceutique, dermatologique, dermo-cosmétique ou vétérinaire de l'invention ou le médicament de l'invention, peuvent être administrés par voie parentérale, telle que par exemple par voie intramusculaire, sous-cutanée, intramédullaire ou intraveineuse ; par injection intrathécale, intraventriculaire, intrapéritonéale ou intraoculaire ; par voie orale, sublinguale, nasale, aérosol, pulmonaire, auriculaire ; par voie topique, cutanée, transdermale, oculaire, rectale, vaginale ; par application sur les ongles ; par toute autre voie permettant une administration localisée, telle que par exemple au niveau d'une tumeur ou d'un tissu.

Selon un mode de réalisation, la composition de l'invention, la composition pharmaceutique, dermatologique, dermo-cosmétique ou vétérinaire de l'invention ou le médicament de l'invention, est formulée par exemple selon l'une des formulations choisies dans le groupe comprenant les formes suivantes : comprimé, comprimé enrobé, comprimé gastrorésistant, cachet, capsule molle, capsule dure, gélule; poudre, pilule, granule, solution, émulsion, suspension, sirop, gouttes oculaires, irrigation sous-gingivales, bain de bouche, chewing-gum, dentifrice, patch, implant, suppositoire, pâte, crème, gel, lotion, lait, pommade, spray, shampoing, vernis, pansement, cathéter, compresse, gaze. Ces formulations peuvent être préparées par les méthodes connues de l'homme du métier.

Selon un mode de réalisation particulier, la composition de l'invention, la composition pharmaceutique, dermatologique, dermo-cosmétique ou vétérinaire de l'invention ou le médicament de l'invention, est formulée sous forme de gel, de préférence d'hydrogel. En particulier, le gel peut être un gel thermosensible, de préférence un hydrogel thermosensible, tel que par exemple le copolymère ((éthylène oxyde)$_{97}$(propylène oxyde)$_{69}$(éthylène oxyde)$_{97}$) connu sous le nom de Pluronic® F127 ou poloxamer P407.

Les formes avantageuses dans le domaine paramédical sont les pansements, les cathéters, les compresses, les gazes, le coton hydrophile, le sérum physiologique, les sprays.

Les formes avantageuses dans le domaine vétérinaire sont les formes orales (comprimés, poudres, capsules molles, capsules dures, gélules, granules, pâtes, solutions, suspensions), injectables (solutions, suspensions), et topiques dont l'action peut être locale ou systémique (sprays, colliers boucles auriculaires, poudres, lotions, pommades, shampoings, patchs, émulsions, laits, gels, crèmes).

Les formes avantageuses dans le domaine cosmétique, de préférence dermo-cosmétique, sont les gels, pâtes, pommades, lotions, crèmes, laits, sticks, shampoings, poudres, aérosols, patchs.

Par ailleurs, la présente invention concerne un médicament comprenant une composition selon l'invention.

Dans un mode de réalisation, la composition de l'invention est utilisée pour désinfecter du matériel ou des équipements médicaux. Dans un mode de réalisation, la composition est utilisée pour imprégner un matériau ou une surface.

Dans un autre mode de réalisation, la composition de l'invention peut être utilisée pour la conservation de substances, en particulier des substances telles que par exemple des produits alimentaires, des boissons, des produits cosmétiques, des produits d'hygiène personnelle, des produits d'hygiène domestique, des peintures ou du bois.

Dans un autre mode de réalisation, la composition de l'invention peut être utilisée pour prévenir ou limiter la formation de biofilms, de préférence de biofilms fongiques. Par « biofilm », il est fait référence à une communauté organisée de cellules fixées à une surface.

*Utilisation antifongique*

La présente invention concerne en outre l'utilisation de la composition de l'invention comme agent antifongique ou dans le traitement d'infections fongiques. Selon un mode réalisation, la composition de l'invention peut également être utilisée pour la prévention d'infections fongiques.

Selon un mode de réalisation, la composition de l'invention utilisée comme agent antifongique ou dans le traitement d'infections fongiques ou pour la prévention d'infections fongiques, comprend du chitosane hydrophobisé de formule générale (I) et un agent antifongique, comme décrit précédemment. La composition comprend en outre une $\alpha$-cyclodextrine.

Selon un mode de réalisation particulier, la composition de l'invention utilisée comme agent antifongique ou dans le traitement d'infections fongiques ou pour la prévention d'infections fongiques, comprend :

- au moins un agent antifongique ;
- du chitosane hydrophobisé de formule générale (Ia) telle que décrite précédemment ;
- une $\alpha$-cyclodextrine, ladite $\alpha$-cyclodextrine étant sous forme de monomère, et formant avec le chitosane hydrophobisé de formule générale (Ia) un complexe d'inclusion non-covalent.

Selon un mode de réalisation, la composition de l'invention est utilisée pour le traitement d'un patient atteint par une infection fongique.

Par « infection fongique », il est fait référence dans la présente invention à toute infection de la peau, des muqueuses ou des phanères à levures (notamment par *Candida sp.*), à moisissures (notamment par *Aspergillus sp.*), à dermatophytes (notamment par *Trichophyton sp.*), à champignons dimorphiques (notamment *Histoplasma sp*) et toutes les infections fongiques invasives (profondes) à levures, moisissures ou champignons dimorphiques.

Selon un autre mode réalisation, la composition de l'invention est utilisée pour traiter un matériau, notamment un matériau tissé ou non tissé, ou une surface contaminés par un microorganisme, de préférence par un champignon.

En particulier, la composition de l'invention peut être utilisée comme agent antifongique ou dans le traitement d'infections fongiques, en relation avec les champignons sélectionnés dans le groupe comprenant *Candida albicans, Aspergillus fumigatus,* les dermatophytes, les champignons dimorphiques et un protozoaire sensible aux antifongiques *Leishmania sp.,* de préférence *Leishmania major.*

Dans un mode de réalisation, le chitosane hydrophobisé de formule (I), de préférence le chitosane hydrophobisé de formule (I) sous forme de particules (y compris les particules de complexes d'inclusion formés de chitosane hydrophobisé de formule (I) et de cyclodextrine), possède des propriétés antifongiques.

Dans un mode de réalisation particulier, la composition de l'invention présente une activité synergique par rapport à l'activité de l'agent antifongique et du chitosane hydrophobisé de formule (I), de préférence du chitosane hydrophobisé de formule (I) sous forme de particules (y compris les particules de complexes d'inclusion formés de chitosane hydrophobisé de formule (I) et de cyclodextrine).

Par « activité synergique », il est fait référence dans la présente invention à l'interaction de deux ou plusieurs agents agissant ensemble de manière positive afin de produire un effet qu'ils ne pouvaient produire seuls.

En particulier, la synergie peut être évaluée selon la méthode recommandée depuis 2003 par l'ASM (American Society for Microbiology) et décrite par Odds (Odds F.C., J. Antimicrobial Chemotherapy, 2003, 52, 1).

Selon un autre mode de réalisation particulier, la composition de l'invention présente une activité additive par rapport à l'activité de l'agent antifongique et du chitosane hydrophobisé de formule (I), de préférence du chitosane hydrophobisé de formule (I) sous forme de particules (y compris les particules de complexe d'inclusion de chitosane hydrophobisé de formule (I) et de cyclodextrine).

Une « activité additive », lors de l'utilisation simultanée de plusieurs agents, correspond à la somme des activités de chaque agent seul.

Dans un mode de réalisation, la quantité d'agent antifongique utilisée dans la composition de l'invention pour obtenir un effet antifongique est inférieure à la quantité habituellement utilisée pour ledit agent antifongique. La possibilité d'utiliser de faibles quantités d'agent antifongique est un avantage de la composition de la présente invention.

Selon un mode de réalisation, la composition antifongique de l'invention peut être administrée seule ou en

combinaison avec un agent actif, tel que par exemple un antimicrobien, un antibiotique, un antifongique, un antiseptique.

Dans un mode de réalisation, la composition antifongique de l'invention est utilisée pour désinfecter du matériel ou des équipements médicaux. Dans un mode de réalisation, la composition est utilisée pour imprégner un matériau ou une surface.

Dans un autre mode de réalisation, la composition antifongique de l'invention peut être utilisée pour la conservation de substances, en particulier des substances telles que par exemple des produits alimentaires, des boissons, des produits cosmétiques, des produits d'hygiène personnelle, des produits d'hygiène domestique, des peintures ou du bois.

Dans un autre mode de réalisation, la composition antifongique de l'invention peut être utilisée pour prévenir ou limiter la formation de biofilms, de préférence de biofilms fongiques. Par « biofilm », il est fait référence à une communauté organisée de cellules fixées à une surface.

## BRÈVE DESCRIPTION DES FIGURES

**Figure 1** est un graphe représentant la viabilité de *Candida albicans* en fonction de la concentration en chitosane hydrophobisé, pour la détermination de la $CI_{50}$ du chitosane hydrophobisé contre *Candida albicans.*

**Figure 2** est un graphe représentant la viabilité de *Candida albicans* en fonction de la concentration en Fungizone®, pour la détermination de la $CI_{50}$ de la Fungizone® contre *Candida albicans.*

**Figure 3** est un graphe représentant la viabilité de *Candida albicans* en fonction de la concentration en chlorhexidine digluconate, pour la détermination de la $CI_{50}$ de la chlorhexidine digluconate contre *Candida albicans.*

**Figures 4A à 4D** est une série d'histogrammes représentant l'effet de la proportion de thiol dans la nanoparticule sur la viabilité de *Trichomonas vaginalis* après incubation de 24 h en présence de nanoparticules; les compositions des nanoparticules PIBCA-[chitosane20/chitosane20-TBA] sont les suivantes : PIBCA-[chitosane20/chitosane20-TBA] 100/0 % m/m (**FIG 4A**), 75/25 % m/m (**FIG 4B)**, 50/50 % m/m (F**IG 4C**), 25/75 % m/m (**FIG 4D**), à différentes concentrations en nanoparticules (12,5, 25, 50 et 100 μg/mL). Les concentrations en nanoparticules testées sont de 12,5, 25, 50 et 100 μg/mL. La viabilité de *Trichomonas vaginalis* a été mesurée dans le milieu de culture sans nanoparticules (C1) et dans le milieu de polymérisation dans nanoparticules (C2). MTZ a été utilisé comme composé référence anti *Trichomonas vaginalis* à une concentration 7 ug/mL. Les valeurs sont les valeurs moyennes de 3 mesures ± sd; * pourcentage par rapport au contrôle $p<0,05$.

**Figure 5** est un histogramme représentant l'effet de nanoparticules de PIBCA-F68 à différentes concentrations sur la viabilité de *Trichomonas vaginalis.* Les concentrations en nanoparticules testées sont 12,5, 25, 50 et 100 μg/mL. La viabilité de *Trichomonas vaginalis* a été mesurée dans le milieu de culture sans nanoparticules (C1) et dans le milieu de polymérisation sans nanoparticules (C2). MTZ a été utilisé comme composé référence anti *Trichomonas vaginalis* à une concentration 7 ug/mL. Les valeurs sont les valeurs moyennes de 3 mesures ± sd; * pourcentage par rapport au contrôle $p<0,05$.

**Figure 6** est un histogramme représentant l'effet de la proportion de chitosane thiolé dans des solutions composées de [chitosane20/chitosane20-TBA] 100/0, 75/25, 50/50 et 25/75 m/m % en solution, sur la viabilité de *Trichomonas vaginalis.* Les solutions ont été testées à une concentration de 12.5 μg/mL en [chitosane20/chitosane20-TBA]. Des résultats identiques ont été obtenus avec des concentrations de 25 μg/mL, 50 μg/mL et 100 μg/mL. La viabilité de *Trichomonas vaginalis* a été mesurée dans le milieu de culture sans nanoparticules (C1) et dans le milieu de polymérisation sans nanoparticules (C2). MTZ a été utilisé comme composé référence anti *Trichomonas vaginalis* à une concentration 7 ug/mL. Les valeurs sont les valeurs moyennes de 3 mesures ± sd; * pourcentage par rapport au contrôle $p<0,05$.

**Figure 7** est une série d'images d'histologie de muqueuses vaginales du porc après 12 h de contact avec les (**A**) nanoparticules composées de PIBCA/[Chitosan20/Chitosan20-TBA] 75/25 % m/m gélifiées avec F127 20 % m/m, (**B**) SVF, (**C**) F127 20 % m/m et (**D**) des particules PIBCA/[Chitosan20/Chitosan20-TBA] 75/25 % m/m non gélifiées. La concentration en nanoparticules est de 20 mg/mL. La barre d'échelle correspond à 0,02 cm. (Grossissement Original x100). (**E**) légère desquamation focale sans défaut du stroma.

**EXEMPLES**

La présente invention se comprendra mieux à la lecture des exemples suivants qui illustrent l'invention.

**Abréviations**

AP : acide palmitique
AO : acide oléique
CD : cyclodextrine
CDCl3 : chloroforme deutéré
$CI_{50}$ : concentration inhibitrice 50
CM : chitosane modifié
$D_2O$ : eau deutérée
Da : unité de masse molaire en dalton, qui correspond au g/L
DCl : acide chlorhydrique deutéré
DDA : degré de désacétylation
DMSO-d6 : diméthylsulfoxyde deutéré
DS : degré de substitution
EDCI : 1-éthyl-3-(3-diméthylaminopropyl) carbodiimide
IBCA : isobutylcyanoacrylate
IR : infrarouge
kDa : kilodalton
kHz : kilohertz
$M_m$ : masse molaire
MOPS : acide 3-(*N*-morpholino)propanesulfonique
MTT : bromure de (3-(4,5-dimethylthiazol-2-yl)-2,5-diphenyltetrazolium)
MHz : Mégahertz
PIBCA : poly(isobutylcyanoacrylate)
RMN : résonance magnétique nucléaire
$\delta$ : déplacement du proton, exprimé en ppm

**Matériel et méthodes**

**$^1$H-RMN** : Les spectres $^1$H-RMN ont été réalisés sur un spectromètre Bruker ARX, 300 MHz ou 500 MHz. Solvants : eau deutérée ($D_2O$) avec 1 % v/v de DCl, chloroforme deutéré (CDCl3), diméthylsulfoxyde deutéré (DMSO-d6). Les spectres sont réalisés à température ambiante avec un champ électromagnétique de 300 MHz. Lorsque le polysaccharide acylé est faiblement soluble ou lorsque la viscosité est élevée, les échantillons peuvent être chauffés jusqu'à 85°C et les spectres sont réalisés avec un champ électromagnétique de 500 MHz.

**$^{13}$C-RMN du solide:** Les spectres $^{13}$C-RMN du solide ont été réalisés sur un spectromètre AVANCE II BRUKER. Des techniques spécialisées de RMN du solide ont été utilisées, notamment la rotation à l'angle magique (MAS) et le transfert de polarisation de $^1$H à $^{13}$C (CP) pour éviter les inconvénients liés aux temps de relaxation parfois très longs à l'état solide. Les mesures ont été effectuées à $\omega L$ = 500 MHz ($^1$H) et 125,77 MHz ($^{13}$C); le temps de contact $^1H/^{13}C$ fixé à 1,5 ms. Les échantillons sont tournés à l'angle magique à une fréquence de 10 kHz, à l'aide un rotor de 4 mm.

**Spectres IR :** Les spectres IR ont été réalisés sur un spectromètre FT/IR-4100, JASCO (ATR-FTIR).

**Microscopie électronique par transmission :** La microscopie électronique par transmission (MET) a été utilisée pour observer les microparticules et nanoparticules à l'aide d'un microscope Jeol 1400 60 kV et d'une caméra.

La MET a également été réalisée à l'aide d'un microscope Philips EM208 et d'une caméra AMT au CCME d'Orsay (Centre Commun de Microscopie Electronique), France. Les préparations sont diluées au 1/100$^{ième}$ dans de l'eau MilliQ®. 3 $\mu$L de ces dilutions sont déposés sur une grille en cuivre avec une membrane de Formvar-Carbone. Après 5 min, une goutte de solution aqueuse à 1% d'acide phosphotungstique est déposée. Après 30 secondes, l'excès de liquide est retiré et l'échantillon est observé au microscope.

**Taille des particules** : La taille des particules a été évaluée par mesure du diamètre hydrodynamique. Les mesures des diamètres hydrodynamiques moyens des nanoparticules et microparticules ont été réalisées à 25°C, avec un Zetasizer nanoséries Nano-ZS90 de la société Malvern instruments SA (Orsay, France) par diffusion quasi-élastique de la lumière. Les échantillons ont été préalablement dilués en prélevant 30 $\mu$L de suspension de nanoparticules ou microparticules et en les diluant dans 1 mL d'eau MilliQ®. La température est équilibrée pendant 5 min avant la mesure.

Le diamètre hydrodynamique moyen est obtenu à partir de trois mesures répétées sur trois dilutions indépendantes.

Les diamètres hydrodynamiques des microparticules ont également été mesurés par un granulomètre laser (MasterSizer 2000) de la société Malvern instruments SA (Orsay, France).

Par ailleurs, le diamètre observé en MET a été mesuré à l'aide du logiciel ImageJ. Le diamètre de 50 nanoparticules est mesuré pour chaque préparation.

**Quantité de soufre** : La quantité de soufre dans les préparations est déterminée par analyse élémentaire à l'aide d'un Analyser LECO SC144 (Service central d'analyse du CNRS, Solaize, France). 10 mg d'échantillon lyophilisé sont chauffés à 1350°C sous flux d'oxygène et le $SO_2$ est détecté par infrarouge.

**Potentiel zeta** : Le potentiel $\zeta$ des particules est mesuré à 25°C à l'aide du Zetasizer nanoseries Nano-ZS (Malvern Instruments, France). 60 $\mu$L de chaque préparation est dilué dans 2 mL d'une solution de NaCl 1mM préalablement filtrée (filtre Millipore Millex-HV Hydrophilic PVDF 0,22 $\mu$m). Le potentiel $\zeta$ moyen est obtenu à partir de trois mesures répétées sur trois dilutions indépendantes.

**Lyophilisation** : Certains dérivés ont été lyophilisés à l'aide d'un lyophilisateur Alpha 1-2 (Avantec, France), pendant 48 heures après avoir congelé les solutions pendant au moins 12 heures.

**Produits** : La chlorhexidine digluconate a été obtenue chez INRESA, Bartenheim, France. La Fungizone® a été obtenue chez Bristol Myers Squibb. Le diméthylformamide anhydre, le dichlorométhane, l'éther diéthylique, l'éthanol, le méthanol, l'ammoniaque, l'acide acétique glacial (98 % (m/m)) proviennent de VWR, France. Le chlorure de *N*-(3-dimethylaminopropyle)-*N*-éthylcarbodiimide (EDCI), l'acide palmitique (99 % (m/m)), l'acide oléique (> 98 % (m/m)), le bicarbonate de sodium, l'hydroxyde de sodium, le chlorure de sodium, le nitrite de sodium ($NaNO_2$), l'acide méthanesulfonique, le chlorure de palmitoyle, le chlorure d'oléoyle, l'eau deutérée, l'acide chlorhydrique deutéré (DCl), le chloroforme deutéré ($CDCl_3$) le diméthylesulfoxyde deutéré (DMSO-$d_6$), la pyridine anhydre, la triéthylamine, le RPMI 1640, le MOPS, le MTT, la ménadione ont été fournis par Sigma-Aldrich Chemical Co, Saint-Quentin Fallavier, France. L'IBCA (isobutylcyanoacrylate, lot 3138/3) provient de chez Henckel Biomedical® (Dublin, Ireland). Le nitrate d'ammonium cérium(IV) (lot 380955/1) a été obtenu chez Fluka® (Saint-Quentin-Fallavier, France). Le Pluronic F68 ou poloxamer 188 (lot 52-0791) provient de chez BASF® (Allemagne). L'hydroxyde de sodium (lot 0400182), le chlorure de sodium (lot N293), l'acide acétique 1N (lot 93067) et l'acide chlorhydrique 37% (lot 92073) ont été fournis par Prolabo® (France). L'acétonitrile (grade HPLC, lot V8D944248D), ainsi que l'acide acétique glacial (lot 1A156051C) viennent de chez Carlo Erba®. L'acide nitrique 63% (lot A018814601) a été obtenu chez Acros Organics® (New Jersey, USA). Le nitrite de sodium NaNO2 (lot 42K3485) viennent de chez Sigma®. Le réactif de Traut (2-iminothiolane HCl) a été synthétisé par le département de chimie organique (Biocis UMR CNRS 8076) de la faculté de Pharmacie de Paris XI (Châtenay-Malabry, France).

Le chitosane de viscosité moyenne et de masse molaire $M_m$=250 kDa, a été fourni par Sigma-Aldrich Chemical Co, Saint-Quentin Fallavier, France.

Les autres chitosanes (20 et 145 kDa) ont été obtenus, sauf mention contraire, après dépolymérisation du chitosane commercial $M_m$=250 kDa selon la technique développée par Huang et al (Pharmaceutical Research, 2004, 21(2), 344): 2 g de chitosane commercial sont solubilisés une nuit dans 100 mL d'acide acétique (6 % v/v), cette solution de chitosane est ensuite dépolymérisée par réaction chimique pendant 1 h avec 10 mL de $NaNO_2$ (85 mg/mL). Le chitosane dépolymérisé est ensuite précipité par augmentation du pH jusqu'à 9 à l'aide d'une solution d'hydroxyde de sodium (4 mol/L) et récupéré par filtration (filtre en verre fritté n°4, sous vide). Il est ensuite solubilisé dans 20 mL d'acide acétique (0,1 mol/L) et dialysé (membrane de dialyse Spectra/Por, MWCO 3500, lot 3228543, Spectrum laboratories Inc®) contre 1 L d'eau distillée deux fois pendant 1h30 puis toute une nuit puis lyophilisé.

La masse molaire du chitosane dépolymérisé est déterminée par viscosimétrie capillaire. Le temps d'écoulement (*t*) dans un microtube $\mu$-Ubbelohde (type 53710/I, n° 1016187, R=0,01022 $mm^2/s^2$, Schott Geräte) de solutions de chitosane dans un mélange acide acétique 0,1 mol/L et NaCl 0,2 mol/L à différentes concentrations (*c* = 0,25/0,50/1,00/1,50/2,00 g/L) est mesuré à 20 °C (bain CT1450 Schott Geräte et système de refroidissement CK100 Schott Geräte) à l'aide d'un viscosimètre AVS400 (Schott Geräte). Pour chaque concentration, le temps d'équilibration est de 5 min et cinq mesures successives sont réalisées. A partir des résultats obtenus la viscosité intrinsèque *[η]* est déduite en prenant l'ordonnée à l'origine de la droite $\frac{t-t_0}{t_0 C} = f(C)$ avec $t_0$ le temps d'écoulement du mélange d'acide acétique 0,1 mol/L et de NaCl 0,2 mol/L. La masse molaire est ensuite calculée en utilisant l'équation de Mark-Houwink-Sakurada $\eta = KM_w^a$, avec *K*=$1{,}81.10^{-6}$ et *a*=0,93.

## 1. Synthèse de chitosanes hydrophobisés

Des chitosanes hydrophobisés ont été synthétisés pour être utilisés dans les compositions de l'invention. En particulier, des chitosanes ont été hydrophobisés par *N*- ou *O*-acylation en présence d'acides gras. Des chitosanes ont également été hydrophobisés par fonctionnalisation par un poly(alkylcyanoacrylate). Avant hydrophobisation, certains chitosanes ont été modifiés par des groupements substitués par une fonction thiol.

### *1.1. Chitosane N-acylé*

*Préparation*

1 g de chitosane ($M_m$=20, 145 ou 250 kDa), dont le degré de désacétylation (DDA) est égal à 85 %, est dissous dans une solution aqueuse d'acide acétique à 1 % v/v (100 mL) diluée avec du méthanol (75 mL). L'acide oléique ou palmitique est dissous dans 10 mL de méthanol et ajouté à la solution de chitosane. Un nombre de mole d'EDCI égal à celui de l'acide gras, est ajouté goutte à goutte au mélange acide gras et chitosane sous agitation magnétique continue. Après 24 heures, le produit est précipité dans un mélange méthanol/ammoniaque 7/3 v/v. Le précipité est filtré sur un filtre en verre fritté et lavé successivement avec l'eau, le méthanol puis l'éther diéthylique. Enfin, le produit est séché sous vide pendant 48 heures.

Cette méthode permet d'obtenir différents types de chitosane *N*-acylé en fonction du type d'acide gras utilisé, du taux de greffage (degré de substitution) et de la masse molaire du chitosane.

*Analyse Infra-rouge*

Les chitosanes hydrophobisés par *N*-acylation ont été analysés par spectrométrie IR. La comparaison avec le spectre du chitosane de départ confirme la formation de la liaison amide.

*Analyse RMN$^1$H*

Pour la caractérisation du chitosane *N*-acylé par RMN du proton, une solution à une concentration égale à 5 g/L est préparée dans de l'eau deutérée ($D_2O$) en présence d'acide chlorhydrique deutéré (DCl). Cette étape permet l'échange des protons labiles des groupements hydroxyles par des atomes de deutérium. Les protons labiles des groupements hydroxyles résonnant tous à la même fréquence, leur échange par des atomes de deutérium permet d'éliminer le signal résiduel de l'eau légère. Afin de diminuer la viscosité, les expériences ont été enregistrées à une température de 85°C avec un nombre d'acquisition et un délai de relaxation de 5 et 1 secondes respectivement.

Les spectres RMN du proton des chitosanes hydrophobisés par *N*-acylation présentent un signal supplémentaire par rapport au spectre du chitosane non hydrophobisé à $\delta$=1,017 ppm. Il correspond au signal du groupe méthyle du bout de la chaîne d'acide gras, confirmant le greffage.

*Caractéristiques des chitosanes hydrophobisés par N-acylation CM1-CM9*

Les caractéristiques des chitosanes hydrophobisés CM1-CM9 sont regroupées dans le tableau 1 ci-dessous. Le degré de substitution a été calculé à partir des résultats de l'analyse élémentaire du chitosane *N*-acylé et du chitosane natif.

**Tableau 1** : Caractéristiques des chitosanes *N*-acylés.

| Chitosane modifié | Acide gras greffé | $M_m$ Chitosane utilisé (kDa) | Degré de substitution (%) |
|---|---|---|---|
| **CM1** | Acide oléique | 250 | 1,19 |
| **CM2** | Acide oléique | 250 | 1,67 |
| **CM3** | Acide oléique | 250 | 7,43 |
| **CM4** | Acide oléique | 145 | 13,47 |
| **CM5** | Acide oléique | 20 | 5,61 |
| **CM6** | Acide oléique | 20 | 6,35 |
| **CM7** | Acide palmitique | 250 | 0,55 |

(suite)

| Chitosane modifié | Acide gras greffé | $M_m$ Chitosane utilisé (kDa) | Degré de substitution (%) |
|---|---|---|---|
| **CM8** | Acide palmitique | 250 | 13,12 |
| **CM9** | Acide palmitique | 250 | 17,01 |

### *1.2. Chitosane O-acylé*

*Préparation*

Le chitosane (250 kDa, 2 g) est dissous dans 20 mL d'acide méthanesulfonique, à température ambiante sous agitation magnétique continue, pendant une heure. Le chlorure d'acide (oléique ou palmitique) est alors introduit dans le milieu réactionnel. Au bout de 5 heures, le mélange est refroidi dans un bain de glace pour arrêter la réaction, un précipité se forme. Le précipité est dialysé pendant 12 heures, puis neutralisé à l'aide du bicarbonate de sodium. Il est ensuite dialysé pendant 48 heures et lyophilisé.
Cette méthode permet d'obtenir différents types de chitosane *O*-acylé en fonction du type de chlorure d'acide gras utilisé, du taux de greffage (degré de substitution) et de la masse molaire du chitosane.

*Analyse Infra-rouge*

Les chitosanes hydrophobisés par *O*-acylation ont été analysés par spectrométrie IR. La comparaison avec le spectre du chitosane de départ confirme la présence des chaines alkyles de l'acide gras.

*Analyse RMN$^1$H*

Les spectres RMN du proton des chitosanes hydrophobisés par *O*-acylation présentent des pics supplémentaires par rapport au spectre du chitosane non hydrophobisé : ceux caractéristiques du groupement CH3 du bout de chaîne à 0,88 ppm et du proton des groupements méthylène CH2 à proximité de la fonction CO à 2,8 ppm.

*Caractéristiques des chitosanes hydrophobisés par O-acylation CM10-CM12*

Les caractéristiques des chitosanes hydrophobisés **CM10-CM12** sont regroupées dans le tableau 2 ci-dessous. Le degré de substitution a été calculé à partir des résultats de l'analyse élémentaire du chitosane *O*-acylé et du chitosane natif.

**Tableau 2** : Caractéristiques des chitosanes *O*-acylés.

| Chitosane modifié | Acide gras greffé | $M_m$ Chitosane utilisé (kDa) | Degré de substitution (%) |
|---|---|---|---|
| **CM10** | Acide oléique | 250 | 1,41 |
| **CM11** | Acide palmitique | 250 | 2,25 |
| **CM12** | Acide palmitique | 250 | 4,64 |

### *1.3. Thiolation du chitosane*

Selon certains aspects de l'invention, le chitosane hydrophobisé comprend des groupements fonctionnalisés par un thiol. L'introduction de ces groupements peut être réalisée sur le chitosane, avant le greffage des groupements hydrophobes.
La thiolation du chitosane, en particulier du chitosane dépolymérisé, peut être réalisée selon la technique développée par Bernkop-Schnürch et al. (International Journal of Pharmaceutics, 2003, vol. 260, n° 2, p. 229-237.) : 1g de chitosane dépolymérisé est solubilisé dans 100 mL d'acide acétique (1% m/v), le pH est ajusté à 6,5 à l'aide d'une solution de NaOH 1 N puis le réactif de Traut est ajouté dans les proportions chitosane/2-iminothiolane : 5/2 (m/m). Après 24h de réaction à température ambiante et sous agitation magnétique, le chitosane-4-thiol-butylamidine (chitosane-TBA) obtenu est dialysé (membrane de dialyse Spectra/Por, MWCO 3500) 8h contre 5L de HCl 5mM, deux fois 8h contre 5L de HCl 5mM/1% NaCl, 8h contre 5L de HCl 5mM et 8h contre 5L de HCl 1mM. Le chitosane-TBA est lyophilisé pendant 48h (lyophilisateur SMH15, Usifroid Procédés Rieutord, Maurepas, France).

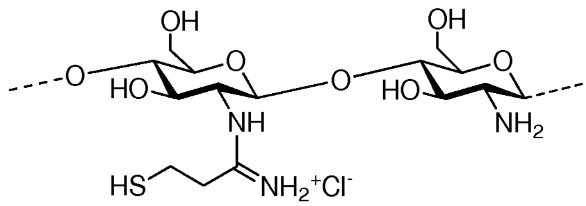

**Chitosane-TBA**

Le chitosane-TBA peut ensuite être greffé par des groupements hydrophobes par N- ou O-acylation comme décrit ci-dessus. Le chitosane-TBA peut également être hydrophobisé par fonctionnalisation par un poly(alkylcyanoacrylate) (cf ci-dessous).

Remarque : Lorsque le chitosane utilisé pour la thiolation a une masse molaire de 20 kDa (« chitosane20 »), le produit obtenu est noté « chitosane20-TBA ».

***1.4. Chitosane fonctionnalisé par un poly(alkylcyanoacrylate)***

Du chitosane a été hydrophobisé par fonctionnalisation par un poly(alkylcyanoacrylate), le poly(isobutylcyanoacrylate) (PIBCA). Le chitosane utilisé pour l'hydrophobisation est un mélange de chitosane dépolymérisé 20 kDa (« chitosane20 ») et de chitosane-TBA obtenu comme décrit ci-dessus (« chitosane20-TBA »).

Le PIBCA-[chitosane/chitosane-TBA] obtenu se présente sous forme de particules.

La fonctionnalisation par le PIBCA peut être réalisée par polymérisation radicalaire en émulsion ou par polymérisation anionique en émulsion.

1.4.1. Préparation par polymérisation radicalaire en émulsion

*Préparation*

Des nanoparticules PIBCA-[chitosane20/chitosane20-TBA] sont préparées par polymérisation radicalaire en émulsion (I. Bravo-Osuna et al., Biomaterials, 2007, vol. 28, n° 13, p. 2233-2243; C. Chauvierre et al., Macromolecules, 2003, vol. 36, n°, p. 6018-6027) : 0,069 g de chitosane 20kDa/chitosane(20kDa)-TBA 75/25 (% m/m) sont dissous dans 4 mL d'acide nitrique 0,2 M. On dissout ensuite dans cette solution de 0 à 4% (m/v) de Pluronic F68. La solution est mise dans un bain à 40°C sous agitation magnétique (plaque Bioblock AM3001K). Après 10 min de bullage d'argon, l'agitation magnétique est augmentée (1000 trs/min) de manière à former un vortex, puis 1mL de solution de nitrate d'ammonium cérium (IV) ($8.10^{-2}$ M dans l'acide nitrique 0,2 M) et 250 μL d'IBCA sont ajoutés à la suite. Le bullage d'argon est maintenu pendant encore 10 min puis la réaction se poursuit pendant 50 minutes. La préparation est ensuite refroidie 5 min dans la glace pillée.

Les nanoparticules sont purifiées par dialyse (membrane de dialyse Spectra/Por, MWCO 100000, Spectrum laboratories Inc.®) contre 1 L d'une solution d'acide acétique 16 μM deux fois 30 min, deux fois 1h puis toute une nuit. Le volume des préparations est ajusté à 8 mL avec de l'eau MilliQ®.

*Taille des nanoparticules*

Le tableau 3 met en évidence que toutes les nanoparticules obtenues sont de taille nanométrique et que la présence de Pluronic F68 dans le milieu de polymérisation lors de la préparation des nanoparticules permet de diminuer leur diamètre hydrodynamique ($D_h$).

**Tableau 3** : Caractérisations physicochimiques des nanoparticules de PIBCA-[chitosane20/chitosane20-TBA] 75/25 % m/m obtenues par polymérisation radicalaire en émulsion en fonction du pourcentage initial du Pluronic F68 présent dans le milieu de polymérization (0, 1, 2, 3 et 4%).

| **Code échantillon** | **$D_h$ (nm)** | **Potentiel ζ (mV)** | **Concentration des groupements -SH[a] (mol/mg)** |
|---|---|---|---|
| Np 0 % | 206 ± 2 | +38 | 1,79 |
| Np 1 % | 134 ± 2 | +31 | 0,58 |
| Np 2 % | 101 ± 1 | +23 | 0,21 |

(suite)

| Code échantillon | $D_h$ (nm) | Potentiel ζ (mV) | Concentration des groupements -SH[a] (mol/mg) |
|---|---|---|---|
| Np 3 % | 93 ± 2 | +14 | 0,06 |
| Np 4 % | 68 ± 1 | +12 | 0,03 |
| Np Control[b] | 103 ± 3 | -7 | 0 |
| (a) iodo titration, (b) nanoparticules de PIBCA non-recouvertes de chitosane stabilisées avec le Pluronic F68. | | | |

*Potentiel ζ des nanoparticules*

Le tableau 3 montre que les nanoparticules PIBCA-[chitosane/chitosane-TBA] 75/25% m/m sont chargées positivement. Ceci est dû à la présence à leur surface d'une couronne de chitosane qui est un polymère chargé positivement (fonctions amines). Au contraire les nanoparticules contrôle ne possédant pas de couronne de chitosane sont chargées négativement.

Les nanoparticules préparées sans Pluronic F68 ont un potentiel ζ d'environ +38mV. La présence de Pluronic F68 lors de la préparation des nanoparticules diminue le potentiel ζ des nanoparticules : il passe de +38mV à +12mV respectivement pour un pourcentage de 0 à 4% de Pluronic F68, on voit donc que les propriétés de surface des nanoparticules ont été modifiées.

*Dosage du soufre*

Le tableau 3 présente également les résultats du dosage du soufre. La présence de Pluronic F68 diminue la quantité totale de soufre dans la préparation (de 0,86% à 0,77% (m/m) respectivement pour 0 et 4% de Pluronic F68 (m/v)) entraînant une modification des propriétés de surface des nanoparticules. Ces résultats pourraient s'expliquer par une compétition entre le chitosane-TBA qui porte les groupements soufrés et le Pluronic F68 lors de la polymérisation.

1.4.2. Préparation par polymérisation anionique en émulsion

*Préparation*

Des nanoparticules PIBCA-[chitosane20/chitosane20-TBA] sont préparées par polymérisation anionique en émulsion selon la méthode de Bravo-Osuna et al (I. Bravo-Osuna et al, Int. J. Pharm. 316(1-2) (2006) 170-175) : 0,069 g de chitosane 20kDa/chitosane(20kDa)-TBA 75/25 % m/m sont dissous dans 5 mL d'acide nitrique 0,2 M. La solution est mise dans un bain à 40°C sous agitation magnétique (plaque Bioblock AM3001K). Après 10 min de bullage d'argon, l'agitation magnétique est augmentée (1000 trs/min) de manière à former un vortex 250 μL d'IBCA sont ajoutés à la suite. Le bullage d'argon est maintenu pendant encore 10 min puis la réaction se poursuit pendant 50 minutes. La préparation est ensuite refroidie 5 min dans la glace pillée.

Les nanoparticules sont purifiées par dialyse comme décrit précédemment.

Divers ratios de chitosane20/chitosane20-TBA ont été mis en oeuvre (100/0, 75/25, 50/50, 25/75 et 0/100 % m/m).

*Caractérisations physicochimiques*

Le tableau 4 met en évidence que les particules obtenues sont de taille nanométrique et sont chargées positivement.

**Tableau 4** : Caractérisations physicochimiques des nanoparticules de PIBCA-[chitosane20/chitosane20-TBA] 75/25% m/m et 100/0% m/m obtenues par polymérisation anionique en émulsion.

| Formulations | $D_h$ (nm) | Potentiel ζ (mV) |
|---|---|---|
| PIBCA/(Chito20/Chito20-TBA) 100/0 % m/m | 195 ± 9 | +43,6 ± 0,3 |
| PIBCA/(Chito20/Chito20-TBA) 75/25 % m/m | 127 ± 1 | +27,1 ± 0,2 |

**2. Particules de chitosane hydrophobisé et de cyclodextrine**

Des microparticules et nanoparticules ont été formées à partir d'α-cyclodextrine et de chitosane hydrophobisé par des acides gras, obtenus comme décrit dans l'exemple 1.1 et 1.2. Les chitosanes hydrophobisés utilisés pour la formation des particules sont listés dans le tableau 3 ci-dessous.

L'a-cyclodextrine ainsi que le chitosane *O*- ou *N*-acylé sont pesés dans un petit flacon. De l'eau distillée est ensuite ajoutée et le mélange est maintenu sous agitation magnétique pendant 3 jours.

Les caractéristiques des particules formées sont présentées dans le tableau 5.

**Tableau 5** : Tailles des particules obtenues à partir du chitosane *N*-acylé ou *O*-acylé.

| Chitosane modifié | %m chitosane amphiphile | %m d'α-CD | %m eau | Taille particules (nm)* |
|---|---|---|---|---|
| **CM8** | 1,0 | 10,0 | 89,0 | 7320 ± 1823 |
| **CM12** | 1,0 | 10,0 | 89,0 | 2090 ± 367 |
| **CM9** | 2,5 | 10,0 | 87,5 | 1416 ± 45 |
| **CM3** | 1 | 10 | 89,0 | 1662 ± 24 |
| *Diamètre hydrodynamique exprimé en volume. | | | | |

**3. Compositions selon l'invention**

Des compositions selon la présente invention ont été préparées.

**Composition 1** : association de Fungizone® et de particules de chitosane hydrophobisé et de cyclodextrine.

| Composés | Type | Concentration |
|---|---|---|
| Fungizone® | agent antifongique | 0,5 % m/v |
| CM3 | chitosane hydrophobisé | 1 % m/v |
| α-cyclodextrine | cyclodextrine | 10 % m/v |

**Composition 2** : association de Chlorhexidine et de particules de chitosane hydrophobisé et de cyclodextrine.

| Composés | Type | Concentration |
|---|---|---|
| chlorhexidine digluconate | agent antifongique | 0,5 % m/v |
| CM3 | chitosane hydrophobisé | 1 % m/v |
| α-cyclodextrine | cyclodextrine | 10 % m/v |

Les compositions 1 et 2 ont été préparées à partir de particules de chitosane hydrophobisé CM3 et de cyclodextrine dans l'eau, comme décrit dans le paragraphe 2 ci-dessus. L'agent antifongique (Fungizone® ou chlorhexidine digluconate) est ensuite ajouté.

**Composition 3 :** encapsulation de la Fungizone® dans des particules de chitosane hydrophobisé et de cyclodextrine.

| Composés | Type | Concentration (% m/v) |
|---|---|---|
| Fungizone® | agent antifongique | 0,5 % m/v |
| CM3 | chitosane hydrophobisé | 1% m/v |
| α-cyclodextrine | cyclodextrine | 10 % m/v |

La composition 3 a été préparée en dissolvant l'agent antifongique dans l'eau puis en ajoutant le chitosane hydrophobisé et la cyclodextrine. Le mélange est maintenu sous agitation magnétique pendant 3 jours. La concentration en agent antifongique qui n'a pas été encapsulé peut être déterminée par dosage dans le surnageant de la préparation.

**Composition 4** : composition comprenant des particules de chitosane hydrophobisé et de cyclodextrine.

| Composés | Type | Concentration (% m/v) |
|---|---|---|
| CM3 | chitosane hydrophobisé | 1% m/v |
| $\alpha$-cyclodextrine | cyclodextrine | 10 % m/v |

La composition 4 a été préparée à partir de particules de chitosane hydrophobisé CM3 et de cyclodextrine dans l'eau, comme décrit dans le paragraphe 2 ci-dessus.

**Compositions 5a à 5d** : compositions comprenant du chitosane hydrophobisé par du PIBCA.

| Composés | Concentration (% m/m) | | | |
|---|---|---|---|---|
| | 5a | 5b | 5c | 5d |
| PIBCA-chitosane20 | 100 | 75 | 50 | 25 |
| PIBCA-chitosane20-TBA | 0 | 25 | 50 | 75 |

Les compositions 5a à 5d ont été préparées comme décrit au paragraphe 1.4.2 ci-dessus.

**4. Activité antifongique des compositions selon l'invention**

L'activité antifongique des compositions de l'invention a été étudiée sur *Candida albicans.* En particulier, des compositions comprenant une association de particules de chitosane hydrophobisé et de cyclodextrine, telles que décrites dans l'exemple 2, et de chlorhexidine digluconate ou de Fungizone® comme agents antifongiques, ont été testées.

Une comparaison de l'activité antifongique des compositions de l'invention a été faite avec l'activité antifongique des constituants pris individuellement. Des effets additifs ou synergiques ont été mis en évidence pour les compositions de l'invention.

***4.1. Matériel***

*Inoculum*

L'inoculum est préparé en prélevant 5 colonies obtenues après 24 h de culture sur milieu gélosé de Sabouraud, chacune d'au moins 1mm de diamètre, et en les mettant en suspension dans 5 mL de NaCl (0,85 %) stérile. La turbidité de chaque suspension est évaluée par la mesure de la densité optique à une longueur d'onde de 530 nm puis ajustée avec une solution isotonique stérile afin d'obtenir une absorbance équivalente à celle obtenue avec le standard turbidimétrique (Mac Farland 0.5).

Ce protocole conduit à l'obtention de suspension dont la concentration est comprise entre $1x10^6$ et $5x10^6$ CFU/mL. Cette suspension est ensuite diluée au 1/1000 avec le milieu de culture pour avoir une concentration finale de $0,5x10^3$ à $2,5x10^3$ CFU/mL.

Une suspension de *Candida albicans* a été préparée dans les conditions décrites ci-dessus.

*Milieu de culture*

Le milieu de culture utilisé pour les tests est le RPMI 1640 sans bicarbonate, 0,2 % de glucose, tamponné à pH7 par du MOPS (0,165M).

***4.2. Méthodes***

*Méthode de mesure de la croissance des levures*

La mesure de la croissance des levures est réalisée par une technique colorimétrique standard faisant appel au sel de tétrazolium MTT (5 mg/mL) (bromure de 3-(4,5-dimethylthiazol-2-yl)-2,5-diphenyl tetrazolium) avec de la ménadione (vitamine K). L'anneau de tétrazolium du MTT est réduit en formazan, par la succinate déshydrogénase mitochondriale des cellules vivantes actives. Ceci forme un précipité dans le milieu de culture de couleur violette. La

quantité de précipité formée est proportionnelle à la quantité de cellules vivantes et à l'activité métabolique de chaque cellule. Il suffit donc après l'incubation des cellules avec du MTT pendant 1h à 37 °C, d'ajouter un agent solubilisant les cristaux de formazan, de centrifuger les plaques 96 puits, et de lire la densité optique du surnageant. Un simple dosage de la densité optique à 570 nm par spectroscopie permet de connaître la quantité relative de cellules vivantes.

*Mesures de $CI_{50}$*

Les mesures des concentrations inhibitrices 50% ($CI_{50}$) sont déterminées selon la méthode américaine approuvée du National Comitee for Clinical Laboratory Standards M27-A3, par microdilution en plaque de 96 puits (Susceptibility test methods : yeast and filamentous fungi. Johnsona et al., in Manual of Clinical Microbiology, 2011, Vol 2, p 2020-2037, ASM press Washington DC).

*Calcul de l'effet d'association : synergie, effet additif, antagonisme ?*

L'effet de l'association de la chlorhexidine digluconate ou de la Fungizone® avec les particules de chitosane hydrophobisé dans les compositions de l'invention est déterminé à partir du calcul du FICI (« fractional inhibitory concentration index ») selon la méthode d'Odds (Odds. F. C. Synergy, antagonism, and what the chequerboard puts between them. Journal of Antimicrobial Chemotherapy, 2003, 52, 1).

Pour déterminer l'effet de l'association de deux molécules A et B, la méthode de Odds comprend les étapes suivantes :

(1) Déterminer la $CI_{50}$ de chaque molécule seule : $CI_{50}^A$ et $CI_{50}^B$ ;
(2) Déterminer les rapports d'association suivants :
A/B = 5/0 (molécule A seule) ; 4/1 ; 3/2 ; 2/3 ; 1/4 ; 0/5 (molécule B seule) ;
(3) Calculer la concentration maximale pour chacune des deux molécules de façon à ce que la $CI_{50}$ soit au centre de 6 dilutions de raison 2, ce qui revient à choisir comme « top concentration », la $CI_{50}$ x 4 ;
(4) Calculer chacune des concentrations en tenant compte des parts de chacune des molécules pour chaque association ;
(5) Pour chacune des associations, faire 6 dilutions de raison 2 (soit 6 dilutions successives au 1/2). Puis, déterminer la $CI_{50}$ de chacune des 4 associations ;
(6) Calculer les concentrations inhibitrices fractionnaires (FIC) pour chaque association :

$$FIC_A = [CI_{50} \text{ de la molécule A quand mélangée à la molécule B}]/[CI_{50}{}^{A}]$$

$$FIC_B = [CI_{50} \text{ de la molécule B quand mélangée à la molécule A}]/[CI_{50}{}^{B}]$$

(7) Calculer la somme des FIC pour chaque association : $\sum FIC = FIC_A + FIC_B$ ;
(8) Calculer l'index des concentrations fractionnaires inhibitrices (FICI) :

$$FICI = \sum FIC / 4 \text{ (i.e. moyenne des } \sum FIC \text{ des 4 associations).}$$

En fonction de la valeur des FICI, il est possible de déterminer s'il y a un effet synergique, additif ou antagoniste :

$$FICI < 0,5 : \text{synergie ;}$$

$$0,5 < FICI < 4 : \text{effet additif ;}$$

$$FICI > 4 : \text{antagonisme.}$$

### *4.3. Activité de particules de chitosane hydrophobisé contre Candida albicans*

*Protocole*

Le test est effectué dans une microplaque de dilution 96 puits (8 lignes A-H, 12 colonnes). 200 µL de milieu de culture sont ajoutés dans les 8 puits de la première colonne (1) et seulement 100 µL dans les puits des autres colonnes (de 2 à 12).

La suspension de particules de chitosane hydrophobisé CM3 préparée selon les conditions décrites dans l'exemple 2 (cf tableau 5) est ajoutée dans les puits de la colonne 1. La concentration du chitosane hydrophobisé dans la suspension de particules est de 1 % m/v. La technique de dilution en cascade des puits de la colonne 1 est utilisée de façon à avoir à chaque fois une dilution au 1/2 en passant d'un puits à l'autre jusqu'à arriver à la colonne 12.

100 µL de suspension de *Candida albicans* (inoculum) sont ajoutés dans chaque puits. La concentration du chitosane hydrophobisé dans les puits de la colonne 1 est de 0,12 %. La microplaque de dilution est incubée à 35°C pendant 24 h.

Le contrôle consiste à procéder de la même façon mais sans rajouter la suspension de chitosane hydrophobisé.

Le pourcentage de viabilité de *Candida albicans* est évalué en utilisant le test au MTT décrit ci-dessus. Dans chaque puits sont rajoutés 10 µL de solution de MTT à une concentration de 5 mg/mL et 10 µL de Menadione à une concentration de 1,72 mg/mL. La plaque est incubée pendant 1 h à 35°C. La microplaque est centrifugée à 1000 rpm pendant 10 minutes. Après centrifugation 100 µL du surnageant est prélevé et la densité optique déterminée par lecture au spectrophotomètre à 570 nm.

Le test a été réalisé au moins trois fois à partir de cultures sur gélose différentes de la souche de *Candida albicans.*

*Résultats*

Les résultats de l'activité des particules de chitosane hydrophobisé contre *Candida albicans* sont présentés sur la Figure 1. La $CI_{50}$ des particules de chitosane hydrophobisé déterminée à partir de la Figure 1 est égale à 0,04 % w/v.

### *4.4. Activité des agents antifongiques contre Candida albicans*

4.4.1. <u>Activité de la Fungizone®</u>

*Protocole*

Préparation de la solution Fungizone® : Un flacon de Fungizone® injectable (50 mg) est reconstitué avec 10 ml d'eau pour préparations injectables. La suspension obtenue contient 5mg/ml d'amphotéricine B et du déoxycholate pour assurer la stabilité de la suspension. La concentration minimale inhibitrice (CMI) observée sur la souche de *Candida albicans* de référence est 0,6 µg/ml dans les conditions de l'essai. La CMI correspond approximativement à la $CI_{90}$%.

Le test est effectué dans une microplaque de dilution 96 puits (8 lignes A-H, 12 colonnes). 200 µL de milieu de culture sont ajoutés dans les puits de la première colonne (1) et seulement 100 µL dans les puits des autres colonnes (de 2 à 12).

La Fungizone® (10 µL), à une concentration de 0,1 mg/mL (elle est diluée au 1/50), est ajoutée dans les puits de la colonne 1. La technique de dilution en cascade des puits de la colonne 1 est utilisée de façon à avoir à chaque fois une dilution au 1/2 en passant d'un puits à l'autre jusqu'à arriver aux puits de la colonne 12.

200 µL de suspension de *Candida albicans* (inoculum) sont ajoutés dans chaque puits. La concentration de la Fungizone® dans les puits de la colonne 1 est de 2,5 µg/mL ou 2,5 mg/L ($2,5x10^{-4}$ % m/v). La microplaque de dilution est incubée à 35°C pendant 24 h.

Le contrôle consiste à procéder de la même façon mais sans rajouter la Fungizone®.

Le pourcentage de viabilité de *Candida albicans* est évalué en utilisant le test au MTT, comme décrit ci-dessus.

*Le test a été réalisé au moins trois fois à partir de cultures sur gélose différentes de la souche de Candida albicans.*
*Résultats*

Les résultats de l'activité de la Fungizone® contre *Candida albicans* sont présentés sur la Figure 2. La $CI_{50}$ de la Fungizone® déterminée à partir de la Figure 2 est égale à 0,3 µg/mL.

4.4.2. Activité de la chlorhexidine digluconate

*Protocole*

Le test est effectué dans une microplaque de dilution 96 puits (8 lignes A-H, 12 colonnes). 200 μL de milieu de culture sont ajoutés dans les puits de la première colonne (1) et seulement 100 μL dans les puits des autres colonnes (de 2 à 12).

La chlorhexidine digluconate (10 μL) à une concentration de 5 mg/mL est ajoutée dans les puits de la colonne 1. La technique de dilution en cascade des puits de la colonne 1 est utilisée de façon à avoir à chaque fois une dilution au 1/2 en passant d'un puits à l'autre jusqu'à arriver aux puits de la colonne 12.

100 μL de suspension de *Candida albicans* (inoculum) sont ajoutés dans chaque puits. La concentration de la chlorhexidine digluconate dans les puits de la colonne 1 est de 2,5 μg/mL (2,5x$10^{-4}$ % m/v). La microplaque de dilution est incubée à 35°C pendant 24 h.

Le contrôle consiste à procéder de la même façon mais sans rajouter la chlorhexidine digluconate.

Le pourcentage de viabilité de *Candida albicans* est évalué en utilisant le test au MTT, dans les mêmes conditions que précédemment.

*Résultats*

Les résultats de l'activité de la chlorhexidine digluconate contre *Candida albicans* sont présentés sur la Figure 3. La $CI_{50}$ de la chlorhexidine digluconate déterminée à partir de la Figure 3 est égale à 0,00068 %.

***4.5. Activité des compositions de l'invention contre Candida albicans***

4.5.1. Composition 1 : Fungizone® associée à des particules de chitosane hydrophobisé et de cyclodextrine

*Protocole*

Le test est effectué dans une microplaque de dilution 96 puits (8 lignes A-H, 12 colonnes). 200 μL de milieu RPMI tamponné sont ajoutés dans les puits de la première colonne (1) et seulement 100 μL dans les puits des autres colonnes (de 2 à 12).

La suspension de particules de chitosane hydrophobisé (50 μL) (cf partie 4.3) ainsi que la Fungizone® (10 μL) à une concentration de 5 mg/mL sont rajoutées dans chaque puits de la colonne 1. La technique de dilution en cascade des particules des puits de la colonne 1 est utilisée de façon à avoir à chaque fois une dilution au 1/2 en passant d'un puits à l'autre jusqu'à arriver aux puits de la colonne 12.

200 μL de suspension de *Candida albicans* (inoculum) sont rajoutés dans chaque puits. La concentration du chitosane hydrophobisé dans les puits de la colonne 1 est de 0,12 %. La concentration de la Fungizone® dans les puits de la colonne 1 est de 1,9 μg/mL (1,9x$10^{-4}$% m/v). La microplaque de dilution est incubée à 35°C pendant 24 h.

Le contrôle consiste à procéder de la même façon mais sans rajouter la suspension de particules de chitosane hydrophobisé ni la Fungizone®.

Le pourcentage de viabilité de *Candida albicans* est évalué en utilisant le test au MTT, dans les mêmes conditions que précédemment.

Le test a été réalisé au moins trois fois à partir de cultures sur gélose différentes de la souche de *Candida albicans.*

*Résultats*

Les résultats de l'activité antifongique de la composition comprenant la Fungizone® et des particules de chitosane hydrophobisé sont détaillés ci-dessous. L'effet de l'association est déterminé par la méthode Odds, détaillée précédemment.

**Étape (1) : $CI_{50}$ (μM) de chaque molécule seule :**

Molécule A : Fungizone® 0,5 % p/v (5 mg/mL)

$$CI_{50}^{A} = 0{,}3\ 10^{-4}\ \%\ p/v\ (0{,}3\ \mu g/mL)$$

Molécule B : Chitosane hydrophobisé formulé en microparticules

$$CI_{50}^{B} = 0{,}04\ \%\ p/v$$

**Étapes (2) à (5) : $CI_{50}$ de chaque association (**

$\mathbf{CI_{50}^{A}\ assoc.1}$ **et** $\mathbf{CI_{50}^{B}\ assoc.1}$ ):

$$CI_{50}^{A}\ assoc.1 = 0{,}028\ 10^{-4}\ \%\ (0{,}028\ \mu g/mL) \qquad CI_{50}^{B}\ assoc.1 = 0{,}0009\ \%$$

$$CI_{50}^{A}\ assoc.2 = 0{,}023\ 10^{-4}\ \%\ (0{,}023\ \mu g/mL) \qquad CI_{50}^{B}\ assoc.2 = 0{,}0023\ \%$$

$$CI_{50}^{A}\ assoc.3 = 0{,}023\ 10^{-4}\ \%\ (0{,}021\ \mu g/mL) \qquad CI_{50}^{B}\ assoc.3 = 0{,}0041\ \%$$

$$CI_{50}^{A}\ assoc.4 = 0{,}023\ 10^{-4}\ \%\ (0{,}022\ \mu g/mL) \qquad CI_{50}^{B}\ assoc.4 = 0{,}0119\ \%$$

**Étape (6) : Concentrations inhibitrices fractionnaires (FIC) :**

$$FIC_{A}^{1} = CI_{50}^{A(assoc.1)}/CI_{50}^{A}\ \text{et}\ FIC_{B}^{1} = CI_{50}^{B(assoc.1)}/CI_{50}^{B}$$

$$FIC_{A}^{1} = 0{,}093 \qquad FIC_{B}^{1} = 0{,}0225$$

$$FIC_{A}^{2} = 0{,}076 \qquad FIC_{B}^{2} = 0{,}0575$$

$$FIC_{A}^{3} = 0{,}070 \qquad FIC_{B}^{3} = 0{,}1025$$

$$FIC_{A}^{4} = 0{,}073 \qquad FIC_{B}^{4} = 0{,}2975$$

**Étape (7) : Calcul de $\sum$ FIC pour chaque association**

$\mathbf{\sum FIC_1 = FIC_A^1 + FIC_B^1}$ :

$$\sum FIC_1 = 0{,}1155$$

$$\sum FIC_2 = 0{,}1335$$

$$\sum FIC_3 = 0{,}1725$$

$$\sum FIC_4 = 0{,}3705$$

**Étape (8): Calcul de FICI = [$\sum FIC_1 + \sum FIC_2 + \sum FIC_3 + \sum FIC_4$] / 4** :

Le résultat de la FICI = 0,198 ce qui indique clairement une synergie entre la Fungizone® et les particules de

chitosane hydrophobisé dans la composition testée.

4.5.2. Composition 2 : Chlorhexidine digluconate associée à des particules de chitosane hydrophobisé et de cyclodextrine

*Protocole*

Le protocole est le même que celui utilisé pour la composition 1 ci-dessus.

La chlorhexidine digluconate est ajoutée à une concentration de 1 %.

La concentration du chitosane hydrophobisé dans les puits de la colonne 1 est de 0,12 %. La concentration de la chlorhexidine digluconate dans les puits de la colonne 1 est de 0,025 %.

*Résultats*

Les résultats de l'activité antifongique de la composition comprenant la chlorhexidine digluconate et des particules de chitosane hydrophobisé sont détaillés ci-dessous. L'effet de l'association est déterminé par la méthode Odds, détaillée précédemment.

**Étape (1) : $CI_{50}$ (µM) de chaque molécule seule :**

Molécule A : Chlorhexidine digluconate 1 %

$$CI_{50}^{A} = 0,00068\ \%$$

Molécule B : Chitosane hydrophobisé formulé en microparticules

$$CI_{50}^{B} = 0,04\ \%$$

**Étapes (2) à (5) : $CI_{50}$ de chaque association (**

$CI_{50}^{A}$ **assoc.1** et $CI_{50}^{B}$ **assoc.1** ) :

$$CI_{50}^{A}\ \text{assoc.1} = 0,000295\ \% \qquad CI_{50}^{B}\ \text{assoc.1} = 0,0047\ \%$$

$$CI_{50}^{A}\ \text{assoc.2} = 0,000235\ \% \qquad CI_{50}^{B}\ \text{assoc.2} = 0,0095\ \%$$

$$CI_{50}^{A}\ \text{assoc.3} = 0,000155\ \% \qquad CI_{50}^{B}\ \text{assoc.3} = 0,0015\ \%$$

$$CI_{50}^{A}\ \text{assoc.4} = 0,000170\ \% \qquad CI_{50}^{B}\ \text{assoc.4} = 0,041\ \%$$

**Étape (6) : Concentrations inhibitrices fractionnaires (FIC) :**

$$FIC_{A}^{1} = CI_{50}^{A(\text{assoc.1})} / CI_{50}^{A} \ \text{ et } FIC_{B}^{1} = CI_{50}^{B(\text{assoc.1})} / CI_{50}^{B}$$

$$FIC_{A}^{1} = 0,434 \qquad FIC_{B}^{1} = 0,1175$$

$$FIC_{A}^{2} = 0,346 \qquad FIC_{B}^{2} = 0,2375$$

$$FIC_{A}^{3} = 0,228 \qquad FIC_{B}^{3} = 0,375$$

(suite)

$$\mathrm{FIC}_A^4 = 0{,}25 \qquad \mathrm{FIC}_B^4 = 1{,}025$$

**Étape (7) : Calcul de ∑ FIC pour chaque association**

$\sum \mathbf{FIC_1} = \mathbf{FIC_A^1} + \mathbf{FIC_B^1}$ :

$$\sum \mathrm{FIC}_1 = 0{,}5515$$

$$\sum \mathrm{FIC}_2 = 0{,}5835$$

$$\sum \mathrm{FIC}_3 = 0{,}603$$

$$\sum \mathrm{FIC}_4 = 1{,}275$$

**Étape (8) : Calcul de FICI = [$\sum$ FIC$_1$ + $\sum$ FIC$_2$ + $\sum$ FIC$_3$ + $\sum$ FIC$_4$] / 4** :

Le résultat de la FICI = 0,753 ce qui indique clairement un effet additif entre la chlorhexidine et les particules de chitosane hydrophobisé dans la composition testée.

**5. Activités antiparasitaires des compositions de l'invention**

***5.1. Activité antiparasitaire contre Leishmania***

L'activité antiparasitaire des compositions de l'invention a été étudiée sur des souches de *Leishmania :*

Les compositions 5a (PIBCA-[chitosane20/chitosane20-TBA] 100/0 m/m %) et 4 (CM3+a-cyclodextrine 1/10% m/v), décrites au point 3 ci-dessus, ont été testées *in vitro* sur trois souches de *Leishmania : Leishmania major* (MHOM/SU/73/5-ASKH/LEM134), *Leishmania tropica* (LRC-L39/LEM2563), *Leishmania braziliensis* (MHOM/BR/75/M2903b). Les $IC_{50}$ ont été mesurées puis comparées au produit de référence l'amphotéricine B pour déterminer l'activité anti-leishmanienne des nanoparticules.

*Matériels*

La culture de promastigote a été réalisée à 26°C dans du milieu M199 (Sigma, Saint-Quentin Fallavier, France) complémenté avec 4,2 mM NaHCO3, 10% de sérum de veau foetal inactivé par chaleur (Invitrogen, Saint-Aubin, France), 40 mM Hepes pH 7,5, 100 μM adénosine et 20 μM hémine (milieu M199 complet).

La culture des amastigotes axéniques a été réalisée à 37°C dans du milieu M199 complet supplémenté avec 200 μM $CaCl_2$ et 200 μM $MgCl_2$.

Les macrophages RAW 264.7 ont été maintenus à 37°C avec 5% de $CO_2$ dans du milieu DMEM (Dulbecco's modified Eagle's médium, Gibco, Saint-Aubin, France) complémenté avec 10% de sérum de veau foetal, inactivé par chaleur (Invitrogen, Saint-Aubin, France), également appelé milieu DMEM complet.

*Méthodes*

Évaluation *in vitro* sur les promastigotes

Le test des compositions sur les prosmatigotes a été conduit comme précédemment décrit (Saint-Pierre-Chazalet et al., J. Antimicrob. Chemother., 2009, 64, 993-1001). Des séries de dilutions ont été réalisées dans 100 μl de milieu M199 complet dans une plaque 96 puits. Des promastigotes en croissance exponentielle ont ensuite été ajoutés à chaque puits à raison de $10^6$/ml dans un volume final de 200 μl. Après 72 h d'incubation à 26°C, la viabilité des promastigotes a été mesurée en utilisant 250 μg/ml de MTT, ou bromure de 3-(4,5-dimethylthiazol-2-yl)-2,5-diphenyl

tetrazolium (Sigma, Saint-Quentin Fallavier, France), qui est réduit en formazan, un produit insoluble, par les mitochondries actives des cellules vivantes. Après 4 h d'incubation à 26°C, le formazan est ensuite solubilisé en ajoutant 100 µl d'isopropanol contenant 40 mM d'HCl dans chaque puits, puis incubé pendant 15 minutes sous agitation. La viabilité des cellules a ensuite été quantifiée en mesurant l'absorbance à 570 nm à l'aide d'un lecteur de plaques (Labsystems Multiskan MS). L'activité des compositions est exprimée en $IC_{50}$. L'amphotéricine B est utilisée comme produit de référence.

Évaluation *in vitro* sur les amastigotes axéniques

Les amastigotes axéniques ont été induits en incubant des promastigotes dans le milieu M199 complet supplementé avec 200 µM de $CaCl_2$ et 200 µM $MgCl_2$ à 37°C avec 5% $CO_2$ pendant 48h. Des dilutions sériées à raison de deux des compositions ont ensuite été réalisées dans 100 µl du même milieu dans des plaques 96 puits. Les amastigotes axéniques ont ensuite été ajoutés dans chaque puits à $10^6$/ml dans 200 µl de volume final. Après 72 h d'incubation à 37°C avec 5% $CO_2$, 20 µl de résazurine à 450 µM ont été ajoutés dans chaque puits, puis incubés à l'obscurité pendant 24 h à 37°C avec 5% $CO_2$. Dans les cellules vivantes, la résazurine est réduite en résofurine. Cette conversion est suivie en mesurant l'absorbance à des longueurs d'ondes spécifiques de la résofurine (570 nm) et de la résazurine (600 nm) en utilisant un lecteur de microplaque (Labsystem Multisken MS). L'activité des compositions est exprimée en $IC_{50}$. L'amphotéricine B est utilisée comme produit de référence.

Évaluation *in vitro* sur les amastigotes intramacrophagiques

L'évaluation des compositions 5a et 4 sur les amastigotes intramacrophagiques a été conduite comme précédemment décrit (Audisio et al., Eur. J. Med. Chem., 2012, 52, 44-50). Les macrophages RAW 264.7 ont été ajoutés dans des puits de plaques 96 puits à raison de 2 x $10^4$ cellules par puits et incubés pendant 24 heures à 37°C avec 5% $CO_2$. Les amastigotes axéniques ont ensuite été induits comme décrit ci-dessus, centrifugés à 2000 g pendant 10 minutes, puis resuspendus dans du milieu DMEM complet, et ajoutés à chaque puits à raison de 3.2 x $10^5$ parasites par puits, pour atteindre un ratio parasite/macrophage de 16 :1. Après 24 h d'infection à 37°C avec 5% $CO_2$, les parasites extracellulaires ont été retirés et 100 µl de milieu DMEM complet contenant les dilutions sériées des compositions ont été ajoutés à chaque puits. Après 48 h de traitement, le milieu de culture est enlevé, puis remplacé par 100µl de réactif DirectPCR Lysis (Euromedex, Souffelweyersheim, France), et trois cycles de congélation-décongélation à température ambiante sont alors réalisés, puis 50 µg/ml de protéinase K sont ajoutés, et une incubation finale à 55°C pendant 4 heures est effectuée pour permettre la lyse complète des cellules. 10 µl de chaque extrait ont ensuite été ajoutés à 40µl de réactif DirectPCR Lysis contenant 0,05% de Sybr Green I (Invitrogen, Saint-Aubin, France). La fluorescence de l'ADN a été mesurée à l'aide du Mastercycler® realplex (Eppendorf, Montesson, France). L'activité des compositions est exprimée sous forme d'$IC_{50}$. L'amphotéricine B est utilisée comme produit de référence.

*Résultats*

Les résultats des tests *in vitro* menés sur les trois souches de *Leishmania* avec la composition 5a et la composition 4 sont présentés dans le tableau ci-dessous. Les résultats avec l'amphotéricine B sont aussi présentés à titre de référence.

| | | **Composition** | | |
|---|---|---|---|---|
| | | **Composition 5a IC50 (ng/µl) ± SEM** | **Composition 4 IC50 (ng/µl) ± SEM** | **Amphotericin B-DOC IC50 (ng/µl) ± SEM** |
| *Leishmania major* | promastigotes | 0,051 ± 0,001 | 43,09 ± 6,97 | 0,015 ± 0,004 |
| | axenic amastigotes | 0,043 ± 0,01 | 11,99 ± 2,88 | 0,038 ± 0,002 |
| | intramacrophagic amastigotes | 5,93 ± 0,43 | 21,86 ± 6,66 | 0,49 ± 0,13 |

(suite)

| | | Composition | | |
|---|---|---|---|---|
| | | Composition 5a IC50 (ng/μl) ± SEM | Composition 4 IC50 (ng/μl) ± SEM | Amphotericin B-DOC IC50 (ng/μl) ± SEM |
| *Leishmania braziliensis* | promastigotes | 0,127 ± 0,007 | 36,38 ± 5,15 | 0,022 ± 0,005 |
| | axenic amastigotes | 0,9 ± 0,31 | 25,32 ± 13,09 | 0,055 ± 0,014 |
| | intramacrophagic amastigotes | 7,49 ± 0,19 | 33,29 ± 12,98 | 0,29 ± 0,09 |
| *Leishmania tropica* | promastigotes | 0,174 ± 0,034 | 87,06 ± 0,69 | 0,023 ± 0,002 |
| | axenic amastigotes | 0,142 ± 0,015 | 32,3 ± 10,86 | 0,049 ± 0,01 |
| | intramacrophagic amastigotes | 3,96 ± 0,74 | 12,05 ± 4 | 0,51 ± 0,07 |

*Interprétation*

Les compositions de l'invention testées sur les différentes souches de *Leishmania* présentent des activités antiparasitaires intéressantes, en particulier la composition 5a qui présente des IC50 inférieures à 10 ng/μl, comparables au produit de référence, et ce, quelle que soit la souche de *Leishmania.*

***5.2. Activité antiparasitaire contre Trichomonas vaginalis***

L'activité antiparasitaire des compositions de l'invention a été étudiée contre *Trichomonas vaginalis* :

5.2.1. Matériel

Les copolymères de qualité pharmaceutique Pluronic® F127 (Poloxamer P407) et Pluronic® F68 (Poloxamer 188) proviennent de BASF ChemTrade GmbH (Ludwigshafen, Allemagne). MTZ, la solution tampon de phosphate (PBS, 0,01 M, pH 7,4 à 25 °C) et tous les autres réactifs utilisés pour la préparation de liquide vaginal simulé (simulated vaginal fluid (SVF)) et pour la thiolation du chitosane proviennent de Sigma-Aldrich (Saint-Quentin Fallavier, France). L'isobutylcyanoacrylate (IBCA) a été offert par Henkel Biomedical (Dublin, Irlande). Le réactif de Traut (2-iminothiolane) a été synthétisé dans le département de chimie organique (Biocis UMR CNRS 8076), Faculté de Pharmacie, Université Paris-Sud (Châtenay-Malabry, France). La souche *Trichomonas vaginalis* (ATCC PRA-98; Taxonoly ID: 412133) a été conservée dans de l'azote liquide contenant 6% de dimethyl sulfoxide comme cryoprotecteur. Cette souche était sensible à MTZ.

Le liquide vaginal simulé (simulated vaginal fluid (SVF)) a été préparé comme précédemment décrit par Owen et Katz (Owen et Katz, Contraception, 1999, 59, 91-95) : NaCl (3,51 g), KOH (1,4 g), $Ca(OH)_2$ (0,22 g), albumine de sérum bovin (0,018 g), acide lactique (2,00 g), acide acetique (1,00 g), glycérol (0,16 g), urée (0,4 g) and glucose (5,00 g) sont ajoutés à 900 mL d'eau distillée contenus dans un bécher et agités mécaniquement jusqu'à dissolution complète. Le pH du mélange est ensuite ajusté à 4,5 par ajout de HCl. Le pH est fixé à 4,5, ce qui correspond à la plage de valeur normale du pH vaginal pré ménopause. Le volume final a été ajusté à 1 L.

Le chitosane hydrosoluble provient de Amicogen (Seoul, Corée). Le poids moléculaire moyen est de 20 000 g/mol selon le fabricant. Ce chitosan est appelé « chitosane20 » ci-après. Le degré de déacétylation est de 92%. L'insertion des groupements thiols sur le chitosane a été menée selon la méthode développée par Bernkop-Schnürch et al., telle que décrite au point 1.3 ci-dessus. Les produits dialysés sont congelés (Christ Alpha 1-4 freeze-dryer. Bioblock Scientific, Illkirch, France) puis stockés à -20°C jusqu'à leur utilisation. Le polymère final est le chitosan-4-thiol-butylamidine, nommé ci-après « chitosane20-TBA ».

5.2.2. Méthode

*Préparation des nanoparticules PIBCA-chitosane*

Les nanoparticules de PIBCA-chitosane et de PIBCA-chitosane thiolé ont été préparées par polymérisation anionique par émulsion selon la méthode décrite par Bravo-Osuna et al., telle que détaillée ci-dessus au point 1.4.2. Les compositions 5a à 5d telles que présentées ci-dessus ont été testées.

Des particules de PIBCA, sans chitosane ni chitosane thiolé, ont été préparées de la même manière en remplaçant le chitosane et le chitosane thiolé par du Pluronic® F68 à hauteur de 1% m/v agissant comme stabilisant. Dans la suite, il est fait référence à ces particules par « nanoparticules de PIBCA/F68 ».

Les suspensions de nanoparticules ont été purifiées par dialyse en utilisant une membrane Spectra/Por® avec une sélection de poids moléculaire de 100000 g/mol, pendant deux cycles de 30 minutes, puis un cycle de 60 minutes et un cycle d'une nuit avec 1 litre de solution d'acide acétique à 16 μM. Les suspensions de nanoparticules sont stockées à 4°C jusqu'à utilisation.

Le milieu de polymérisation, utilisé comme milieu contrôle (contrôle 2, noté C2) pour l'évaluation de l'activité *anti-Trichomonas vaginalis,* a été préparé de la même manière que précédemment décrit, mais sans IBCA, ni chitosane ni Pluronic® F68, puis purifié dans les mêmes conditions que pour la purification des nanoparticules.

*Caractérisations physico-chimiques des nanoparticules*

Le diamètre hydrodynamique moyen des nanoparticules et leur distribution de taille ont été déterminés par la moyenne Z obtenue à 20°C par dispersion de lumière quasi-élastique avec un Zetasizer Nanoseries (Malvern Instruments Ltd. UK). L'angle de dispersion a été fixé à 173° et 60 μL de chaque échantillon a été dilué dans 2 mL d'une solution d'acide acétique à 0,16 μM (Millex, SLAP 0225, Millipore, France). Le potentiel Zeta des nanoparticules a été mesuré avec Zetasizer Nanoseries (Malvern Instruments Ltd. UK). La dilution des suspensions (1:33 (v/v)) a été conduite avec une solution de NaCl (1 mM). Chaque expérience a été menée en 3 exemplaires.

*Préparation des hydrogels*

Des hydrogels ont été préparés comme décrit précédemment (Bouchemal K. et al., Aka-Any-Grah A. et al., Zhang M. et al.) : des hydrogels de Pluronic® F127 (20 m/m %) ont été obtenus en ajoutant progressivement sous agitation le Pluronic® F127 en poudre dans une eau maintenue à 4°C. L'hydrogel contenant les nanoparticules a été préparé en dispersant lentement sous agitation magnétique le Pluronic® F127 en poudre (20 m/m %) directement dans la dispersion de nanoparticule maintenue à 4°C jusqu'à la dissolution complète du polymère. Les nanoparticules sont composées de PIBCA-[chitosane20/chitosane20-TBA] 75/25 m/m % (composition 5a à 5d). La concentration en nanoparticules dans l'hydrogel est de 20 mg/mL.

*Évaluation de l'activité anti Trichomonas vaginalis des compositions*

*Trichomonas vaginalis* est cultivé axeniquement à 35°C dans un milieu trypticase-extrait de levure-maltose (TYM) (Diamond L.S. et al., J. Parasitol., 1957, 43(4), 488-490) enrichi avec 10% de sérum de cheval décomplémenté (Gibco, France) ; la souche est repiquée tous les deux jours (Camuzat-Dedenis B. et al., Eur. J. Med. Chem., 2001, 36(10), 837-842).

Les tubes de culture contenant le milieu TYM frais enrichi avec 10% de sérum de cheval seul (contrôle 1, noté Cl) ou ceux avec les compositions testées, sont inoculés avec le parasite à raison de 2 x $10^5$ protozoaires dans 3 mL. Les compositions testées sont les compositions de PIBCA-[chitosane20/chitosane20-TBA] présentant différentes proportions de chitosane et de chitosane thiolé, à savoir 100/0, 75/25, 50/50 et 25/75 m/m %. Les concentrations en nanoparticules étudiées sont 12,5, 25, 50 et 100 μg/mL.

Les résultats sont comparés entre (i) les nanoparticules de PIBCA/F68 ; (ii) les solutions composées de mélanges de PIBCA-[chitosane20/ chitosane20-TBA] présentant différentes proportions de chitosane et chitosane thiolé, à savoir 100/0, 75/25, 50/50 et 25/75 m/m %, (iii) le milieu de polymérisation (contrôle 2, noté C2). Le MTZ est utilisé comme produit de référence anti *Trichomonas vaginalis* à une concentration létale de 7 μg/mL.

Les tubes sont incubés pendant 24 heures à 35°C et le nombre de parasites par millilitre dans chaque tube est déterminé microscopiquement avec un hémocytomètre (Kova® Glasstic® Slide 10, Hycor Biomedical, Etats-Unis). Les expériences sont reproduites trois fois.

Les résultats sont exprimés sous forme de pourcentage d'inhibition de la culture cellulaire en fonction du contrôle 1.

Pour l'analyse statistique, le test de Student a été appliqué. Le seuil de significativité est $p < 0,05$.

*Évaluation ex vivo de toxicité des compositions sur les muqueuses vaginales du porc*

Les expériences ont été menées sur des femelles de porc ((INRA Jouy en Josas, France) pesant entre 60 et 63 kg en moyenne. Les animaux jeûnent pendant 24 heures mais ont libre accès à l'eau courante. Toutes les expériences sur les animaux obéissent à la Directive Européenne (DE/86/609/CEE) et ont été conduites en conformité avec l'autorisation No. 78-16 du Ministère de l'agriculture français. Les porcs sont sacrifiés par injection en intraveineuse (20 mL) d'une surdose de sodium phénobarbital (Dolethal, Vetoquinol Laboratory, Lure, France) et la muqueuse vaginale est prélevée sur une longueur de 10 cm. La muqueuse est ensuite placée dans le liquide vaginal simulé (simulated vaginal fluid (SVF)) et mise à -20°C immédiatement après le sacrifice de l'animal puis stockée à cette température jusqu'à la prochaine utilisation. Il a été montré que les muqueuses porcines pouvaient être gelées pour leur conservation sans que la couche de mucus ne soit affectée (Squier CA. et al.). Les échantillons sont découpés par pièces de 1 $cm^2$ à l'aide de ciseaux de chirurgien afin d'obtenir des tissus vaginaux intactes puis décongelés avant l'expérience à température ambiante dans du SVF fraîchement préparé.

La muqueuse est ensuite placée dans les cellules de Franz et le compartiment collecteur est rempli de SVF (11.2 mL). Environ 0.5 mL de chaque composition est uniformément déposé sur la muqueuse. La surface de contact est de 1 $cm^2$. La composition étudiée est composée de PIBCA-[chitosane20/chitosane20-TBA] 75/25 m/m% gélifiée avec du Pluronic® F127. Les contrôles sont : le SVF, du Pluronic® F127 et les nanoparticules sans hydrogel. La concentration en nanoparticules est de 20 mg/mL et la concentration en Pluronic® F127 est de 20 m/m %.

Les cellules de Franz sont refermées et placées dans un bain thermostaté à 37°C. Après 12 heures en contact avec les compositions, les liquides récepteurs et donneurs sont collectés. Les tissus sont fixés dans FineFix (Milestone, Italy), incorporés dans de la paraffine et coupés en fines lamelle de 4 μm. La coloration avec hematoxilin-eosin-saffranin a été faite avant les analyses histologiques. Les images sont été obtenues avec un microscope Axiophot Zeiss (Allemagne) connecté avec une caméra digitale (PCO, Allemagne). La totalité des échantillons a été scannées avec un scanner de diapositive (Nikon Super Coolscan 8000) adapté à l'histopathologie (Groupe Régional d'études sur le cancer, Caen, France).

5.2.3. Résultats

*Caractérisation physico chimique des nanoparticules*

Les nanoparticules de PIBCA-[chitosane20/chitosane20-TBA] ont été obtenues par polymérisation anionique par émulsion par polymérisation du monomère d'isobutylcyanoacrylate. Ces nanoparticules sont composées d'un coeur hydrophobe de PIBCA alors que l'enveloppe externe est formée par une couche d'un mélange de chitosane et de chitosane thiolé avec différentes proportions en thiol 100/0, 75/25, 50/50 et 25/75 m/m %. Les nanoparticules sans chitosane sont stabilisées avec du Pluronic® F68. Les diamètres moyens hydrodynamiques des nanoparticules varient entre 185 et 210 nm :

| Composition des nanoparticules (% m/m) | | $D_h$ (nm) | Potentiel ζ (mV) |
|---|---|---|---|
| PIBCA-[chitosane20/chitosane20-TBA] | 100/0 | 185 ± 2 | +39.6 ± 0.6 |
| | 75/25 | 211 ± 4 | +34.3 ± 1.0 |
| | 50/50 | 196 ± 2 | +39.0 ± 0.4 |
| | 25/75 | 192 ± 2 | +40.4 ± 0.8 |

Les mesures de mobilité en électrophorèse montrent que les potentiels zeta des nanoparticules PIBCA-chitosane sont positifs et varient entre +34.3 to +40.4 mV. Ces valeurs diffèrent légèrement selon la composition de l'enveloppe de la nanoparticule.

*Évaluation in vitro de l'activité anti-Trichomonas vaginalis des compositions*

L'activité *anti-Trichomonas vaginalis* des nanoparticules composées de PIBCA-[chitosane20/chitosane20-TBA] 75/25 m/m % a été étudiée après incubation de 24 heures avec *Trichomonas vaginalis.* L'activité anti *Trichomonas vaginalis* des nanoparticules est comparée avec l'activité de la molécule de référence, MTZ, utilisé à une concentration létale de 7 μg/mL.

Les nanoparticules testéesmontrent un puissant effet anti-*Trichomonas vaginalis* à une concentration de 100 μg/mL (Figures 4A-4D). La variation de la proportion en chitosane thiolé n'affecte pas l'activité *anti-Trichomonas vaginalis.*

En effet, une activité anti-*Trichomonas vaginalis* similaire a été observée avec les compositions présentant des ratios différents (PIBCA-[chitosane20/chitosane20-TBA] 100/0, 75/25, 50/50 et 25/75 m/m %) (Figure 4A à 4D).

Aucune activité *anti-Trichomonas vaginalis* n'a été observée sur les nanoparticules de PIBCA/F68 (i.e. sans chitosane) (Figure 5), ni avec des solutions de chitosane (i.e. sans PIBCA) (Figure 6). De plus, aucune activité n'a été observée avec le milieu de polymérisation (Figures 5 et 6).

*Évaluation ex vivo de la toxicité des compositions sur les muqueuses vaginales de porc*

L'histotoxicité des nanoparticules PIBCA-[chitosane20/chitosane20-TBA] 75/25 m/m% gélifiée avec du Pluronic® F127 20 m/m % a été étudiée ex vivo sur les muqueuses vaginales du porc. Les résultats de la Figure 7A montrent que l'épithélium était normal avec une architecture complétement conservée en comparaison avec l'expérience menée dans du SVF (Figure 7B) et du Pluronic® F127 20 m/m % (Figure 7C). En présence de particules non gélifiées, l'épithélium a une architecture normale avec une légère desquamation focale sans anomalies du stroma (Figure 7D). Les ulcérations sont absentes de tous les échantillons de muqueuses et le stroma de base est dépourvu de cellules inflammatoires ce qui confirme l'absence d'une toxicité significative.

## Revendications

**1.** Composition comprenant :

- au moins un agent antifongique ;
- du chitosane hydrophobisé de formule générale (I) :

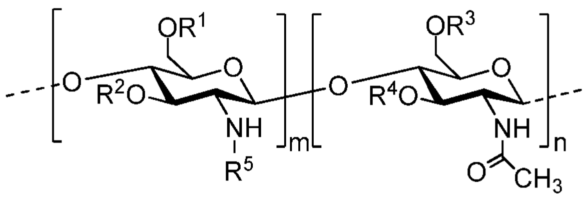

dans laquelle

- $R^1$, $R^2$, $R^3$, $R^4$ et $R^5$ sont identiques ou différents, et représentent :
  - un atome d'hydrogène ;
  - un groupe hydrophobe ;
  - un groupe substitué par une fonction thiol ;

  sous réserve que au moins l'un de $R^1$, $R^2$, $R^3$, $R^4$ et $R^5$ est un groupe hydrophobe ;
- n et m représentent chacun indépendamment un entier compris entre 1 et 3000, de préférence entre 5 et 1500, plus préférentiellement entre 25 et 100, sous réserve que le pourcentage de m par rapport à m+n soit supérieur à 50% ;

- une $\alpha$-cyclodextrine, ladite $\alpha$-cyclodextrine étant sous forme de monomère et formant avec le chitosane hydrophobisé de formule générale (I) un complexe d'inclusion non-covalent.

**2.** Composition selon la revendication **1,** dans laquelle le chitosane hydrophobisé de formule générale (I) est tel que le groupe hydrophobe est sélectionné parmi :

◦ un groupe de formule $-COR^6$, dans lequel $R^6$ représente :

- un groupe alkyle linéaire ou ramifié, comprenant de 1 à 1000 atomes de carbone, de préférence de 1 à 20 atomes de carbone, plus préférentiellement les groupes $-(CH_2)_{14}-CH_3$ ou $-(CH_2)_{16}-CH_3$ ;
- un groupe alcényle linéaire ou ramifié, comprenant de 2 à 1000 atomes de carbone, de préférence de 2 à 20 atomes de carbone, et comprenant au moins une double liaison C=C, de préférence de 1 à 4 doubles

liaisons, de préférence les groupes $-(CH_2)_7-CH=CH-CH_2-(CH_2)_7-CH_3$ ou $-(CH_2)_7-CH=CH-(CH_2)_7-CH_3$ ;

◦ un poly(alkylcyanoacrylate) dans lequel l'alkyle est linéaire ou branché, comprenant de 1 à 12 atomes de carbone, de préférence l'alkyle est un méthyle, propyle, isopropyle, butyle, isobutyle, pentyle, isopentyle, hexyle, isohexyle, heptyle, isoheptyle, octyle, isooctyle, nonyle, isononyle, décyle, isodécyle, undécyle, iso-undécyle, dodécyle, isododécyle et dérivés, plus préférentiellement le poly(alkylcyanoacrylate) est le poly(isobutylcyanoacrylate).

3. Composition selon la revendication **1** ou la revendication **2,** dans laquelle le chitosane hydrophobisé de formule générale (I) est tel que le groupe substitué par une fonction thiol est un alkyle substitué par une fonction thiol, un alcényle substitué par une fonction thiol ou un groupe $-C(=NH_2^+X^-)-(CH_2)_q-SH$ dans lequel X représente un halogène, de préférence Cl, et q représente un entier allant de 1 à 10, de préférence 1, 2, 3, 4, ou 5.

4. Composition selon l'une quelconque des revendications **1** à **3,** dans laquelle l'α-cyclodextrine est de formule générale (II) :

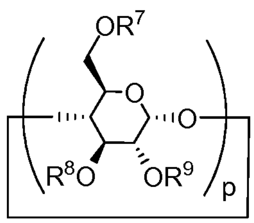

dans laquelle

• p est égal à 6,
• $R^7$, $R^8$ et $R^9$ sont identiques ou différents, de préférence identiques, et représentent chacun indépendamment un atome d'hydrogène ; un groupement alkyle comportant 1 à 4 atomes de carbone, de préférence choisi dans le groupe comprenant méthyle, éthyle, propyle, isopropyle ; $-NH_2$, $-NH_3^+$, ou $-SO_4^{2-}$ ; de préférence, $R^7$, $R^8$ et $R^9$ représentent un atome d'hydrogène ou un groupement méthyle.

5. Composition selon l'une quelconque des revendications **1** à **4,** dans laquelle l'agent antifongique est sélectionné dans le groupe comprenant :

- les polyènes, tels que par exemple l'amphotéricine B, la candicidine, la filipine, l'hamycine, la natamycine, la nystatine, la rimocidine ;
- les composés imidazolés tels que par exemple le bifonazole, le butoconazole, le clotrimazole, l'éconazole, le fenticonazole, l'isoconazole, le kétoconazole, le miconazole, l'omoconazole, l'oxiconazole, le sertaconazole, le sulconazole, le tioconazole ;
- les composés triazolés, tels que par exemple l'albaconazole, le fluconazole, l'isavuconazole, l'itraconazole, le posaconazole, le ravuconazole, le terconazole, le voriconazole ;
- les composés thiazolés, tels que par exemple l'abafungine ;
- les allylamines, telles que par exemple l'amorolfine, la butenafine, la naftifine, la terbinafine ;
- les échinocandines, telles que par exemple l'anidulafungine, la caspofungine, la micafungine ;
- l'acide benzoïque, la cérulénine, le ciclopirox, l'olamine, la flucytosine, la 5-fluorocytosine, la griséofulvine, l'haloprogine, le polygodial, le tolnaftate, l'acide undecylénique, le crystal violet ; ou
- la chlorhexidine, la polyvinylpyrrolidone (PVP) iodée, le chlorure de benzalkonium, le chlore.

6. Composition pharmaceutique, dermatologique, dermo-cosmétique ou vétérinaire comprenant la composition selon l'une quelconque des revendications **1** à **5,** en association avec un excipient pharmaceutiquement, dermatologiquement, dermo-cosmétiquement ou vétérinairement acceptable.

7. Composition selon la revendication **6,** administrable par voie parentérale, intramusculaire, sous-cutanée, intramédullaire ou intraveineuse ; par injection intrathécale, intraventriculaire, intrapéritonéale ou intraoculaire ; par voie orale, sublinguale, nasale, aérosol, pulmonaire, auriculaire; par voie topique, cutanée, transdermale, oculaire, rectale, vaginale ; par application sur les ongles ; par toute autre voie permettant une administration localisée.

**8.** Composition selon la revendication **6** ou la revendication **7,** sous forme de comprimé, comprimé enrobé, comprimé gastrorésistant, cachet, capsule molle, capsule dure, gélule; poudre, pilule, granule, solution, émulsion, suspension, sirop, gouttes oculaires, irrigation sous-gingivales, bain de bouche, chewing-gum, dentifrice, patch, implant, suppositoire, pâte, crème, gel, lotion, lait, pommade, spray, shampoing, vernis, pansement, cathéter, compresse, gaze.

**9.** Médicament comprenant une composition selon l'une quelconque des revendications **1** à **5.**

**10.** Composition selon l'une quelconque des revendications **1** à **5** pour son utilisation comme agent antifongique ou dans le traitement d'infections fongiques.

**11.** Procédé d'association pour la préparation d'une composition selon l'une quelconque des revendications **1** à **5,** comprenant les étapes suivantes :

(a) du chitosane hydrophobisé de formule générale (I) selon la revendication **1,** est placé dans un solvant, de préférence l'eau, en présence d'une α-cyclodextrine, sous agitation, pour former une suspension de particules ;
(b) au moins un antifongique est ajouté dans la suspension.

**12.** Procédé d'encapsulation pour la préparation d'une composition selon l'une quelconque des revendications **1** à **5,** comprenant les étapes suivantes :

(a) au moins un agent antifongique est dissous dans un solvant, de préférence l'eau ;
(b) du chitosane hydrophobisé de formule générale (I) selon la revendication **1,** et une α-cyclodextrine, sont ajoutés dans la solution ;

le mélange est agité pour former une suspension de particules de chitosane et de cyclodextrine, encapsulant l'agent antifongique.

## Patentansprüche

**1.** Zusammensetzung, umfassend:

➢ mindestens ein Fungizid;

➢ hydrophobisiertes Chitosan der allgemeinen Formel (I):

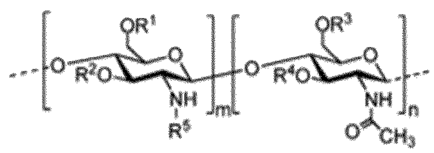

wobei

• $R^1$, $R^2$, $R^3$, $R^4$ und $R^5$ identisch oder unterschiedlich sind und darstellen:

- ein Wasserstoffatom;
- eine hydrophobe Gruppe;
- eine von einer Thiolfunktion substituierte Gruppe;

sofern mindestens eins von $R^1$, $R^2$, $R^3$, $R^4$ und $R^5$ eine hydrophobe Gruppe ist;
• n und m jeweils unabhängig eine Ganzzahl zwischen 1 und 3000, vorzugsweise zwischen 5 und 1500, noch bevorzugter zwischen 25 und 100, inklusive darstellen, sofern der Prozentsatz von m in Bezug auf m+n größer als 50 % ist;

➢ ein α-Cyclodextrin, wobei das α-Cyclodextrin in Form von Monomer vorliegt und mit dem hydrophobisierten Chitosan der allgemeinen Formel (I) einen nicht kovalenten Inklusionskomplex bildet.

**2.** Zusammensetzung nach Anspruch 1, wobei das hydrophobisierte Chitosan der allgemeinen Formel (I) derart ist, dass die hydrophobe Gruppe ausgewählt ist aus:

◦ einer Gruppe der Formel $-COR^6$, wobei $R^6$ darstellt:

▪ eine lineare oder verzweigte Alkylgruppe, umfassend 1 bis 1000 Kohlenstoffatome, vorzugsweise 1 bis 20 Kohlenstoffatome, noch bevorzugter die Gruppen $-(CH_2)_{14}-CH_3$ oder $-(CH_2)_{16}-CH_3$;
▪ eine lineare oder verzweigte Alkenylgruppe, umfassend 2 bis 1000 Kohlenstoffatome, vorzugsweise 2 bis 20 Kohlenstoffatome, und umfassend mindestens eine Doppelbindung C=C, vorzugsweise 1 bis 4 Doppelbindungen, vorzugsweise die Gruppen$-(CH_2)_7-CH=CH-CH_2-(CH_2)_7-CH_3$ oder $-(CH_2)_7-CH=CH-(CH_2)_7-CH_3$;

o einem Poly(alkylcyanoacrylat), wobei das Alkyl linear oder verzweigt ist, umfassend 1 bis 12 Kohlenstoffatome, wobei das Alkyl vorzugsweise ein Methyl, Propyl, Isopropyl, Butyl, Isobutyl, Pentyl, Isopentyl, Hexyl, Isohexyl, Heptyl, Isoheptyl, Octyl, Isooctyl, Nonyl, Isononyl, Decyl, Isodecyl, Undecyl, Isoundecyl, Dodecyl, Isododecyl und Derivate, noch bevorzugter das Poly(alkylcyanoacrylat) das Poly(isobutylcyanoacrylat) ist.

**3.** Zusammensetzung nach Anspruch 1 oder Anspruch 2, wobei das hydrophobisierte Chitosan der allgemeinen Formel (I) derart ist, dass die von einer Thiolfunktion substituierte Gruppe ein Alkyl ist, das von einer Thiolfunktion substituiert ist, ein Alkenyl, das von einer Thiolfunktion substituiert ist oder eine Gruppe $-C(=NH_2^+X^-)-(CH_2)_q-SH$, wobei X ein Halogen darstellt, vorzugsweise Cl, und q eine Ganzzahl von 1 bis 10, vorzugsweise 1, 2, 3, 4 oder 5, darstellt.

**4.** Zusammensetzung nach einem der Ansprüche 1 bis 3, wobei das $\alpha$-Cyclodextrin die allgemeine Formel (II) hat:

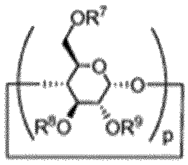

wobei

• p gleich 6 ist,
• $R^7$, $R^8$ und $R^9$ identisch oder unterschiedlich, vorzugsweise identisch sind und jeweils unabhängig ein Wasserstoffatom darstellen; wobei eine Alklygruppe 1 bis 4 Kohlenstoffatome aufweist, vorzugsweise ausgewählt aus der Gruppe, die Methyl, Ethyl, Propyl, Isopropyl umfasst; $-NH_2$, $-NH_3^+$ oder $-SO_4^{2-}$; wobei vorzugsweise $R^7$, $R^8$ und $R^9$ ein Wasserstoffatom oder eine Methylgruppe darstellen.

**5.** Zusammensetzung nach einem der Ansprüche 1 bis 4, wobei das Fungizid aus der Gruppe ausgewählt ist, umfassend:

- die Polyene wie beispielsweise das Amphotericin B, das Candicidin, das Filipin, das Hamycin, das Natamycin, das Nystatin, das Rimocidin;
- die Imidazolverbindungen wie beispielsweise das Bifonazol, das Butoconazol, das Clotrimazol, das Econazol, das Fenticonazol, das Isoconazol, das Ketoconazol, das Miconazol, das Omoconazol, das Oxiconazol, das Sertaconazol, das Sulconazol, das Tioconazol;
- die Triazolverbindungen wie beispielsweise das Albaconazol, das Fluconazol, das Isavuconazol, das Itraconazol, das Posaconazol, das Ravuconazol, das Terconazol, das Voriconazol;
- die Thiazolverbindungen wie beispielsweise das Abafungin;
- die Allylamine wie beispielsweise das Amorolfin, das Butenafin, das Naftifin, das Terbinafin;
- die Echinocandine wie beispielsweise das Anidulafungin, das Caspofungin, das Micafungin;
- die Benzoesäure, das Cerulenin, das Ciclopirox, das Olamin, das Flucytosin, das 5-Fluorcytosin, das Griseofulvin, das Haloprogin, das Polygodial, das Tolnaftat, die Undecylensäure, das Kristallviolett; oder
- das Chlorhexidin, das jodierte Polyvinylpyrrolidon (PVP), das Benzalkoniumchlorid, das Chlor.

**6.** Pharmazeutische, dermatologische, dermokosmetische oder Veterinär-Zusammensetzung, umfassend die Zusammensetzung nach einem der Ansprüche 1 bis 5, in Kombination mit einem pharmazeutisch, dermatologisch, dermokosmetisch oder veterinärmäßig akzeptablen Hilfsstoff.

**7.** Zusammensetzung nach Anspruch 6, verabreichbar auf parenteralem, intramuskulärem, subkutanem, intramedullärem oder intravenösem Weg; durch intrathekale, intraventrikuläre, intraperitoneale oder intraokuläre Injektion; auf oralem, sublingualem, nasalem Weg, als Aerosol, auf pulmonalem, aurikulärem Weg; auf topischem, kutanem, transdermalem, okularem, rektalem, vaginalem Weg; durch Auftragen auf die Nägel; durch jeden anderen Weg, der eine lokale Verabreichung erlaubt.

**8.** Zusammensetzung nach Anspruch 6 oder Anspruch 7 in Form einer Tablette, einer umhüllten Tablette, einer gastroresistenten Tablette, einer Pastille, Weichkapsel, Hartkapsel, einer Gelkapsel; eines Pulvers, einer Pille, eines Granulats, einer Lösung, Emulsion, Suspension, eines Sirups, von Augentropfen, einer subgingivalen Spülung, Mundwasser, Kaugummis, einer Zahncreme, eines Patchs, Implantats, Zäpfchens, einer Paste, Creme, eines Gels, einer Lotion, Milch, Pomade, eines Sprays, Shampoons, Lacks, Verbands, Katheters, einer Kompresse, einer Gaze.

**9.** Arzneimittel, umfassend eine Zusammensetzung nach einem der Ansprüche 1 bis 5.

**10.** Zusammensetzung nach einem der Ansprüche 1 bis 5 für ihre Verwendung als Fungizid oder bei der Behandlung von Pilzinfektionen.

**11.** Kombinationsverfahren für die Zubereitung einer Zusammensetzung nach einem der Ansprüche 1 bis 5, umfassend die folgenden Schritte:

(a) Platzieren von hydrophobisiertem Chitosan der allgemeinen Formel (I) nach Anspruch 1 in ein Lösungsmittel, vorzugsweise Wasser, bei Anwesenheit eines $\alpha$-Cyclodextrins, unter Rühren, um eine Partikelsuspension zu bilden;
(b) Hinzufügen von mindestens einem Fungizid in die Suspension.

**12.** Kapselverfahren für die Zubereitung einer Zusammensetzung nach einem der Ansprüche 1 bis 5, umfassend die folgenden Schritte:

(a) Lösen mindestens eines Fungizids in einem Lösungsmittel, vorzugsweise Wasser;
(b) Hinzufügen von hydrophobisiertem Chitosan der allgemeinen Formel (I) nach Anspruch 1 und eines $\alpha$-Cyclodextrins in die Lösung;

wobei das Gemisch gerührt wird, um eine Suspension von Chitosan- und Cyclodextrin-Partikeln zu bilden, welche das Fungizid einkapseln.

## Claims

**1.** A composition, comprising:

(i) at least one antifungal agent
(ii) hydrophobised chitosan with the general formula (I):

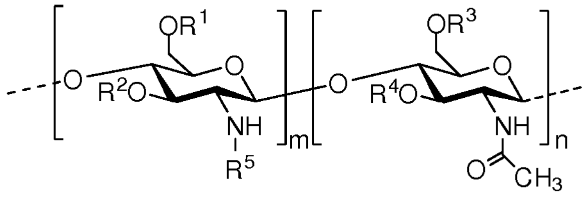
(I)

wherein
$R^1$, $R^2$, $R^3$, $R^4$ and $R^5$ are identical or different, and each represents:

- a hydrogen atom;
- a hydrophobic group; or

- a group substituted by a thiol function;

provided that at least one of $R^1$, $R^2$, $R^3$, $R^4$ and $R^5$ is a hydrophobic group;
n and m each independently represent an integer between 1 and 3000, preferably between 5 and 1500, more preferably between 25 and 100, provided that the percentage of m with respect to m+n is greater than 50%; and
(iii) an α-cyclodextrin, said α-cyclodextrin being in the form of a monomer, et forming with the hydrophobised chitosan of general formula (I) a non-covalent inclusion complex.

2. The composition according to claim **1,** wherein the hydrophobised chitosan of general formula (I) is such that the hydrophobic group is selected from:

a group with the formula $-COR^6$, wherein $R^6$ represents:

a linear or branched alkyl group, comprising from 1 to 1000 carbon atoms, preferably 1 to 20 carbon atoms, more preferably the groups $-(CH_2)_{14}-CH_3$ or

$-(CH_2)_{16}-CH_3$ ;

a linear or branched alkenyl group, comprising from 2 to 1000 carbon atoms, preferably from 2 to 20 carbon atoms and comprising at least one double bond C=C, preferably from 1 to 4 double bonds, preferably the groups $-(CH_2)_7-CH=CH-CH_2-(CH_2)_7-CH_3$ or ;$-(CH_2)_7-CH=CH-(CH_2)_7-CH_3$ and

a poly(alkylcyanoacrylate) wherein the alkyl is linear or branched, comprising from 1 to 12 carbon atoms, preferably the alkyl is methyl, propyl, isopropyl, butyl, isobutyl, pentyl, isopentyl, hexyl, isohexyl, heptyl, isoheptyl, octyl, isooctyl, nonyl, isononyl, decyl, isodecyl, undecyl, isoundecyl, dodecyl, isododecyl and derivatives, more preferably the poly(alkylcyanoacrylate) is the poly(isobutylcyanoacrylate).

3. The composition according to claim **1 or claim 2,** wherein the hydrophobised chitosan of general formula (I) is such that the the group substituted by a thiol function is an alkyl substituted by a thiol function, an alkenyl substituted by a thiol function or a group $-C(=NH_2^+X^-)-(CH_2)_q-SH$ wherein X represents a halogen, preferably Cl, and q represents an integer ranging from 1 to 10, preferably 1, 2, 3, 4 and 5.

4. The composition according to anyone of claims **1 to 3,** wherein the α-cyclodextrin is with the general formula (II):

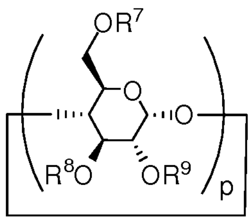

wherein

• p is equal to 6,
• $R^7$, $R^8$ and $R^9$ are identical or different, preferably identical, and each independently represent a hydrogen atom; an alkyl group comprising 1 to 4 carbon atoms, preferably chosen from the group comprising methyl, propyle, isopropyle, $-NH_2$, $-NH_3^+$ or $SO_4^{2-}$ ; preferably , $R^7$, $R^8$ and $R^9$ represent a hydrogen atom or a methyl group..

5. The composition according to anyone of claims **1 to 4,** wherein the antifungal agent is selected from the group consisting of:

- polyenes, such as for example amphotericin B, candicidin, filipin, hamycin, natamycin, nystatin, rimocidine;
- imidazole compounds such as, for example, bifonazole, butoconazole, clotrimazole, econazole, fenticonazole, isoconazole, ketoconazole, miconazole, omoconazole, oxiconazole, sertaconazole, sulconazole, tioconazole;
- triazole compounds such as, for example,, albaconazole, fluconazole, isavuconazole, itraconazole, posaconazole, ravuconazole, terconazole, voriconazole;

- thiazole compounds such as, for example,, abafungine;
- allylamines such as, for example,, amorolfine, butenafine, naftifine, terbinafine;
- echinocandins such as, for example, anidulafungine, caspofungine, micafungine;
- benzoic acid, cerulenin, ciclopirox, olamine, flucytosine, 5-fluorocytosine, griseofulvine, haloprogine, polygodial, tolnaftate, undecylenic acid, crystal violet; and
- chlorhexidine, polyvinylpyrrolidone (PVP) iodine, benzalkonium chloride, chlorine.

**6.** A pharmaceutical, dermatological, dermo-cosmestic or veterinary composition comprising the composition according to anyone of claims **1 to 5,** in association with a pharmaceutically, dermatologically, dermo-cosmetically or veterinarily acceptable excipient.

**7.** The composition according to claim **6,** wherein the composition is formulated to be administrable by at least one of parenteral, intramuscular, subcutaneous, intramedullary or intravenous route; by intrathecal injection, intraventricular, intraperitoneal or intraocular route; by oral, sublingual, nasal, aerosol, pulmonary, ear route; by topical, cutaneous, transdermal, ocular, rectal, vaginal route; by application on the nails; or by any other route that allows for a localised administration.

**8.** The composition according to claim **6 or claim 7,** wherein the composition is in the form of a tablet, coated tablet, gastro-resistant tablet, tablet, soft capsule, hard capsule, hard-shelled capsule; powder, pill, granule, solution, emulsion, suspension, syrup, eye drops, subgingival irrigation, mouth bath, chewing gum, toothpaste, patch, implant, suppository, paste, cream, gel, lotion, milk, ointment, spray, shampoo, varnish, plaster, catheter, compress, or gauze.

**9.** A medicament comprising the composition according to anyone of claim **1 to 5.**

**10.** The composition according to anyone of claims **1 to 5, formulated** for use as an antifungal agent or in the treatment of fungal infections.

**11.** Process of association for preparing the composition of anyone of claims **1 to 5,** comprising the following steps:

(a) placing a hydrophobised chitosan with the general formula (I), in a solvent, preferably water, in the presence of an $\alpha$-cyclodextrin, under stirring, in order to form a suspension of particles;
(b) adding at least one antifungal to the suspension.

**12.** Process of encapsulation for preparing the composition of anyone of claims **1 to 5,** comprising the following steps:

(a) dissolving at least one antifungal agent in a solvent, preferably water;
(b) adding a hydrophobised chitosan with the general formula (I) according to claim 1 and an $\alpha$-cyclodextrin to the solution;

stirring the mixture to form a suspension of particles of chitosan and of cyclodextrin, encapsulating said antifungal agent.

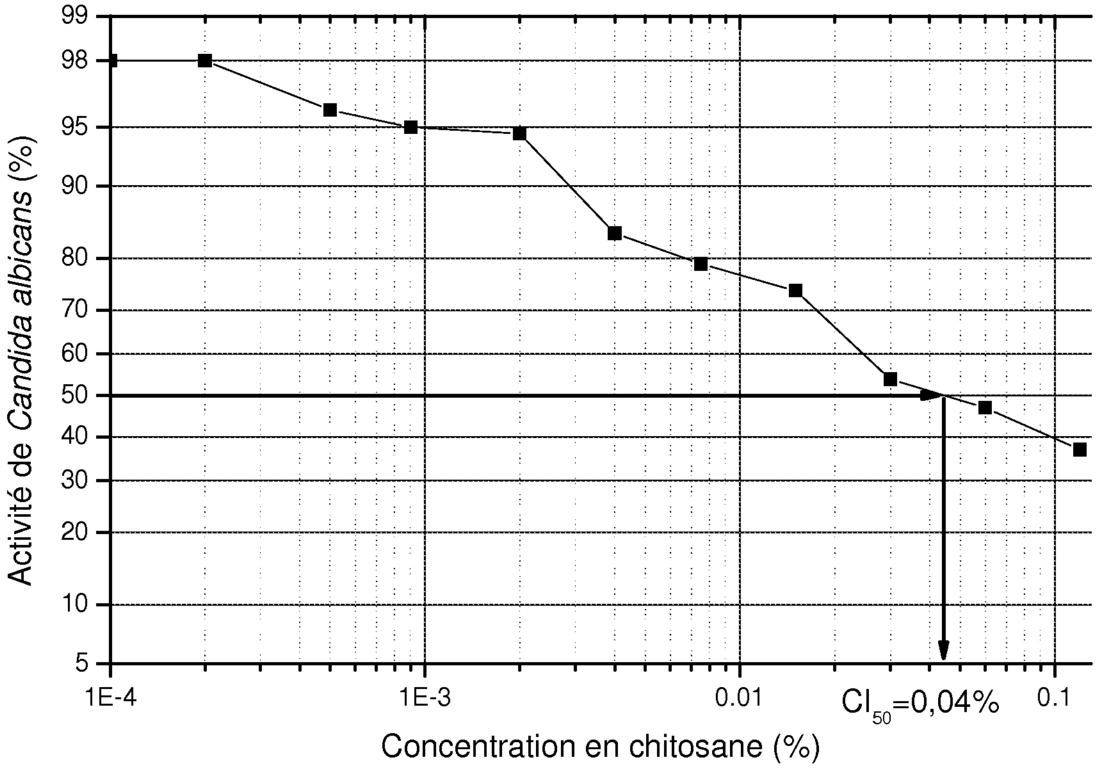
99
98
95
90
80
70
60
50
40
30
20
10
5
Activité de *Candida albicans* (%)
1E-4
1E-3
0.01
$CI_{50}$=0,04%
0.1
Concentration en chitosane (%)

FIG. 1

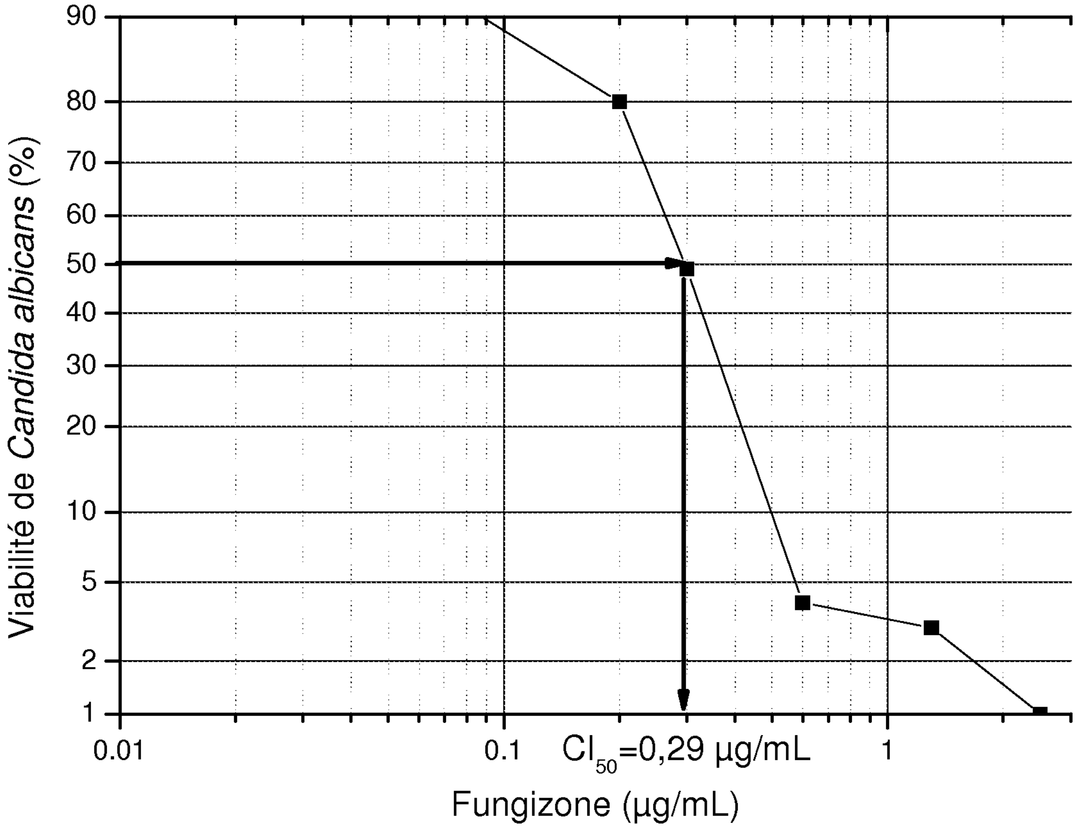
Viabilité de Candida albicans (%)
90
80
70
60
50
40
30
20
10
5
2
1
0.01
0.1
1
CI50=0,29 µg/mL
Fungizone (µg/mL)

**FIG. 2**

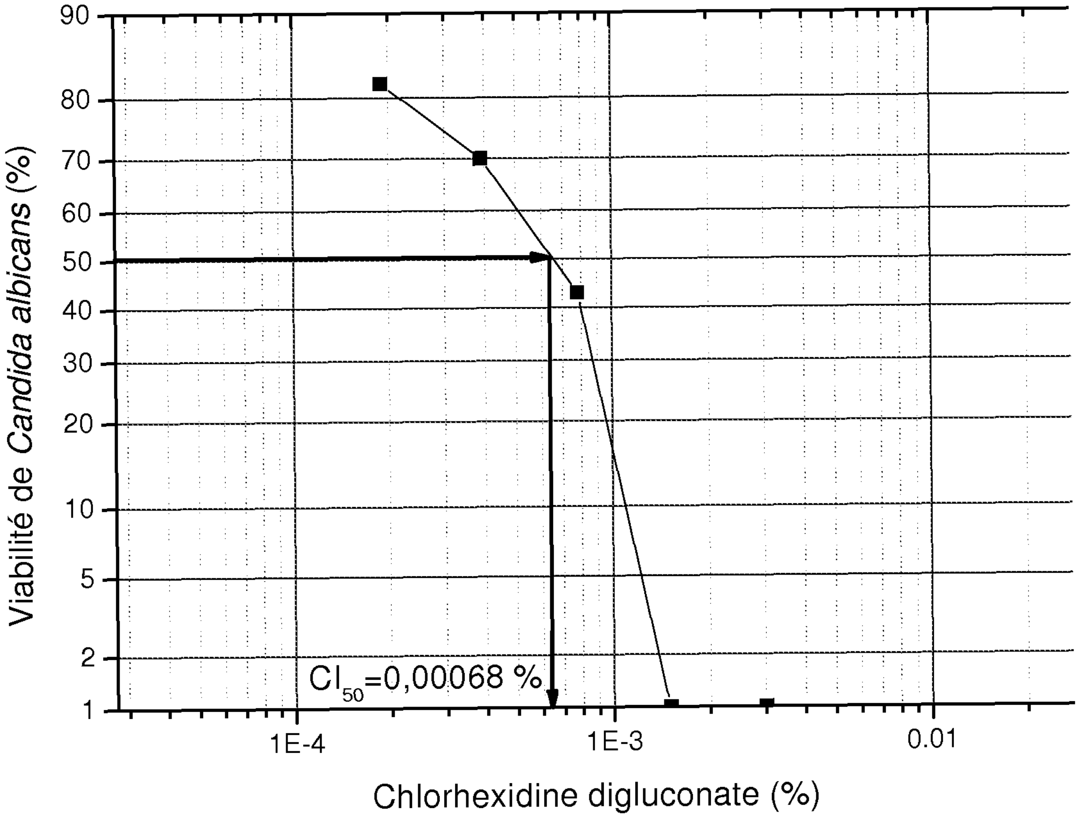
90
80
70
60
50
40
30
20
10
5
2
1
Viabilité de *Candida albicans* (%)
CI50=0,00068 %
1E-4
1E-3
0.01
Chlorhexidine digluconate (%)

FIG. 3

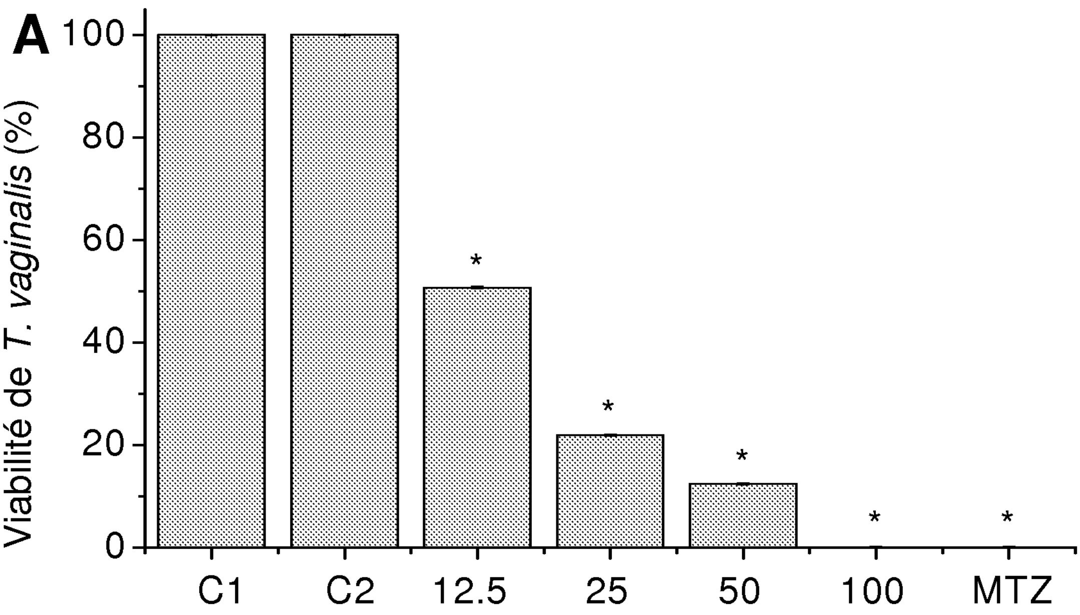
A
Viabilité de T. vaginalis (%)
100
80
60
40
20
0
*
C1
C2
12.5
25
50
100
MTZ

**FIG. 4A**

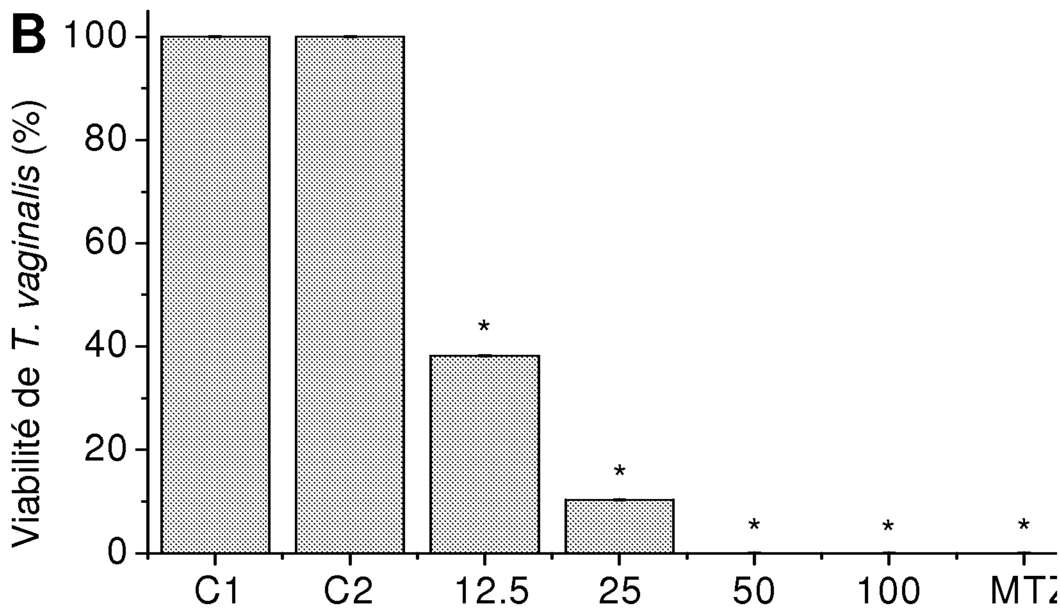
B
Viabilité de T. vaginalis (%)
100
80
60
40
20
0
*
C1
C2
12.5
25
50
100

**FIG. 4B**

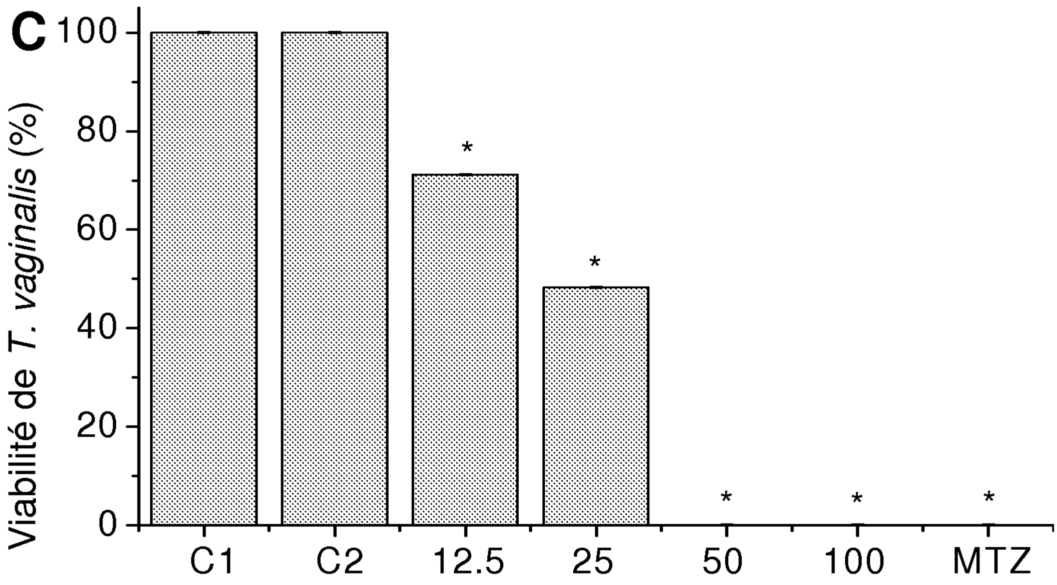
C
Viabilité de T. vaginalis (%)
100
80
60
40
20
0
*
*
*
*
*
C1
C2
12.5
25
50
100
MTZ

FIG. 4C

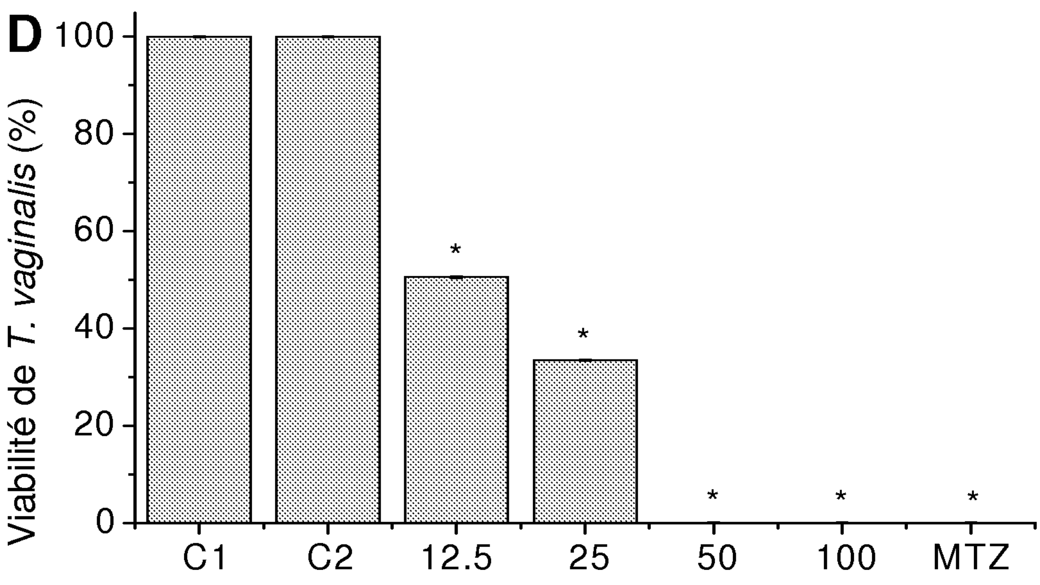
D
Viabilité de T. vaginalis (%)
100
80
60
40
20
0
*
*
*
*
*
C1
C2
12.5
25
50
100
MTZ

FIG. 4D

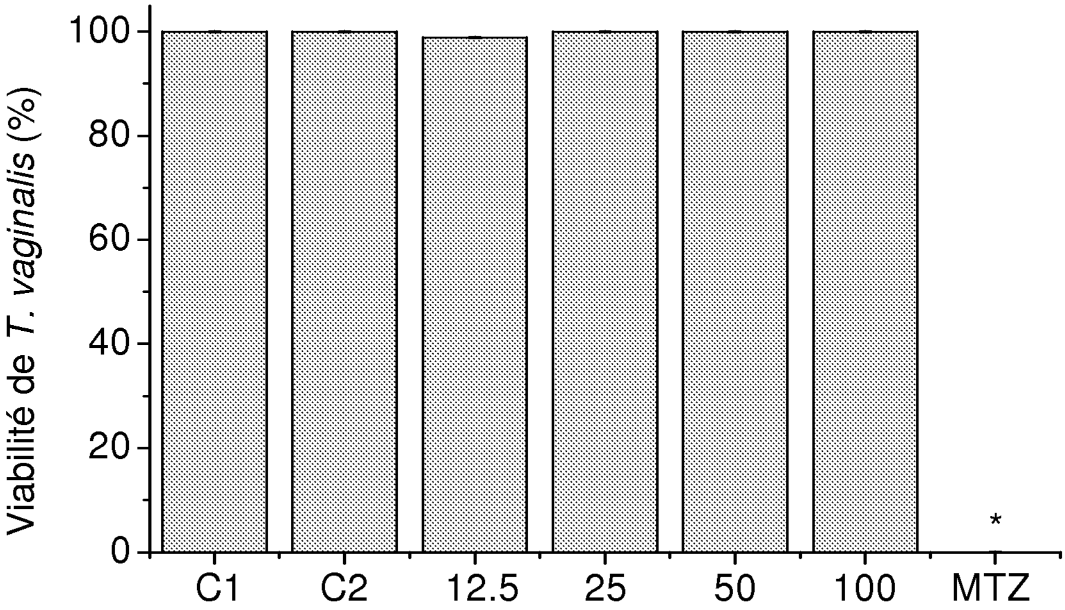
Viabilité de T. vaginalis (%)
100
80
60
40
20
0
*
C1
C2
12.5
25
50
100
MTZ

FIG. 5

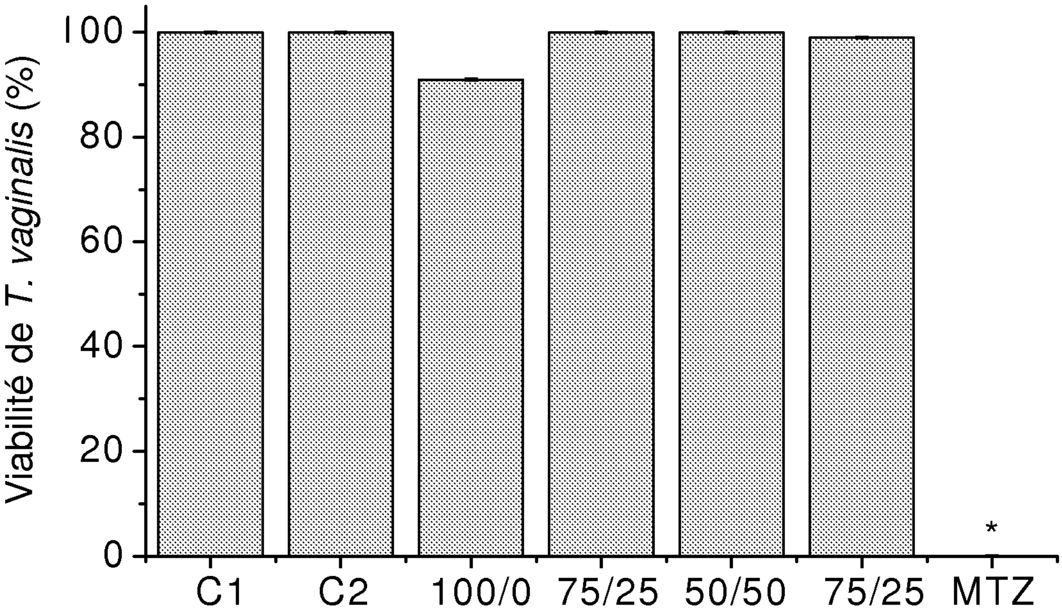
Viabilité de T. vaginalis (%)
100
80
60
40
20
0
*
C1
C2
100/0
75/25
50/50
75/25
MTZ

FIG. 6

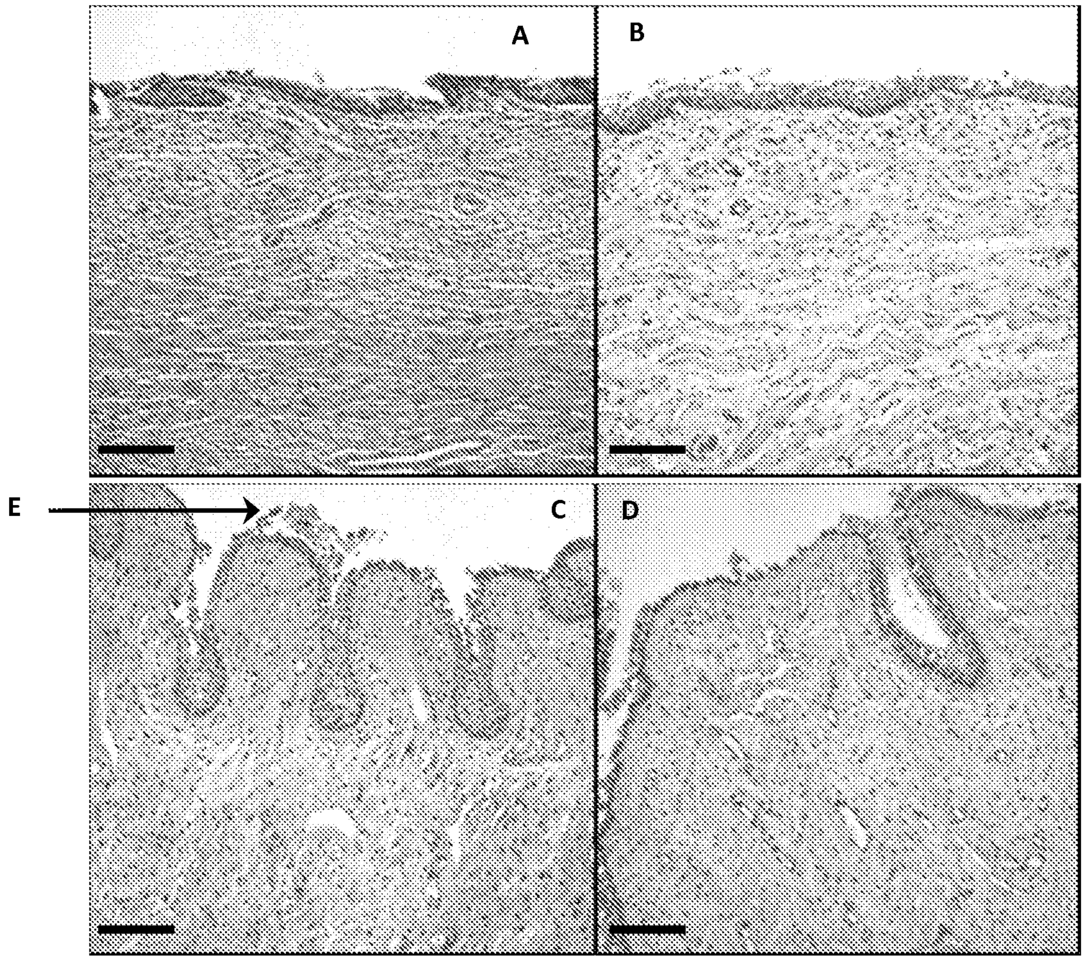


FIG. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **VIEIRA et al.** *J. Nanobiotech.,* 2008, vol. 6, 6
- **ALBASARAH et al.** *J. Pharmacy Pharmacol.,* 2010, vol. 62, 821-828
- **LIMPEANCHOB et al.** *Naresuan Univ. J.,* 2006, vol. 14 (2), 27-34
- **GHARIB et al.** *DARU,* 2011, vol. 19 (5), 351-355
- **GILANI et al.** *J. Pharm Sci,* 2011, vol. 100 (1), 252-259
- **MOAZENI et al.** *Daru J. Pharm. Sc.,* vol. 20 (85), 1-9
- **ODDS F.C.** J. Antimicrobial Chemotherapy. American Society for Microbiology, 2003, vol. 52, 1
- **HUANG et al.** *Pharmaceutical Research,* 2004, vol. 21 (2), 344
- **BERNKOP-SCHNÜRCH et al.** *International Journal of Pharmaceutics,* 2003, vol. 260 (2), 229-237
- **I. BRAVO-OSUNA et al.** *Biomaterials,* 2007, vol. 28 (13), 2233-2243
- **C. CHAUVIERRE et al.** *Macromolecules,* 2003, vol. 36, 6018-6027
- **I. BRAVO-OSUNA et al.** *Int. J. Pharm.,* 2006, vol. 316 (1-2), 170-175
- Susceptibility test methods : yeast and filamentous fungi. **JOHNSONA et al.** Manual of Clinical Microbiology. ASM press, 2011, vol. 2, 2020-2037
- **ODDS. F. C.** Synergy, antagonism, and what the chequerboard puts between them. *Journal of Antimicrobial Chemotherapy,* 2003, vol. 52, 1
- **SAINT-PIERRE-CHAZALET et al.** *J. Antimicrob. Chemother.,* 2009, vol. 64, 993-1001
- **AUDISIO et al.** *Eur. J. Med. Chem.,* 2012, vol. 52, 44-50
- **OWEN ; KATZ.** *Contraception,* 1999, vol. 59, 91-95
- **DIAMOND L.S. et al.** *J. Parasitol.,* 1957, vol. 43 (4), 488-490
- **CAMUZAT-DEDENIS B. et al.** *Eur. J. Med. Chem.,* 2001, vol. 36 (10), 837-842